# EUROPEAN PATENT SPECIFICATION

(11) **EP 2 000 150 B1**
(45) Date of publication and mention of the grant of the patent: **13.07.2016**
(21) Application number: 07739321.3
(22) Date of filing: 22.03.2007
(51) Int. Cl.: A61K 41/00, A61K 9/08, A61K 33/24, A61K 47/32, A61K 47/34, A61K 47/36, A61P 35/00, A61P 43/00, C01G 23/047

(54) **TITANIUM OXIDE COMPLEX PARTICLE, DISPERSION SOLUTION OF THE PARTICLE, AND PROCESS FOR PRODUCTION OF THE PARTICLE**
TITANOXIDKOMPLEXTEILCHEN, DISPERSIONSLÖSUNG DES TEILCHENS UND HERSTELLUNGSVERFAHREN FÜR DAS TEILCHEN
PARTICULES COMPLEXES D'OXYDE DE TITANE, DISPERSION DES PARTICULES ET PROCÉDÉ POUR LA PRODUCTION DES PARTICULES

(30) Priority: 24.03.2006 JP 2006083512; 20.09.2006 JP 2006254912
(43) Date of publication of application: 10.12.2008
(73) Proprietor: TOTO LTD., Kitakyushu-shi Fukuoka 802-8601 (JP)
(72) Inventor: KANEHIRA, Koki, Kitakyushu-shi, Fukuoka 802-8601 (JP); SONEZAKI, Shuji, Kitakyushu-shi, Fukuoka 802-8601 (JP); OGAMI, Yumi, Kitakyushu-shi, Fukuoka 802-8601 (JP); NAKAMURA, Tomomi, Kitakyushu-shi, Fukuoka 802-8601 (JP)
(74) Representative: Grünecker Patent- und Rechtsanwälte PartG mbB
(86) International application number: PCT/JP2007/055878
(87) International publication number: WO 2007/122956

(56) References cited:
- WO-A1-93/26019
- WO-A1-2004/087577
- WO-A1-2004/087765
- WO-A1-2005/120590
- WO-A1-2006/016800
- WO-A1-2006/025627
- JP-A- 2004 059 393
- JP-A- 2005 289 660
- JP-A- 2005 314 409
- JP-A- 2006 102 586
- US-A1- 2004 258 762

## Description

The present invention provides titanium oxide composite particles comprising titanium oxide particles having surface modified with a hydrophilic polymer and, a dispersion liquid thereof. The titanium oxide composite particles can be rendered cytotoxic e.g. upon exposure to ultrasonic waves or, ultraviolet light and thus can be utilized as a cell killer for killing cells such as cancer cells, or as an ultrasonic cancer treatment enhancer for enhancing ultrasonic cancer treatment by irradiating an affected part with ultrasonic waves.

### BACKGROUND ART

Titanium oxide is said to have an isoelectric point at a pH value around 6. Accordingly, the titanium oxide particles disadvantageously cause coagulation in an aqueous solvent around a neutral pH, making it very difficult to dispersing the titanium oxide particles homogeneously. In view of this, various attempts have been made for homogeneously dispersing titanium oxides particles in an aqueous dispersion medium.

It is known that PEG (polyethylene glycol) is added as a dispersant to improve the dispersibility of titanium oxide particles in a dispersion medium (see patent document 1 (Japanese Patent Laid-Open No. 307524/1990), patent document 2 (Japanese Patent Laid-Open No. 60651/2002)) and patent document 3 (WO 2005/120590 A1).

On the other hand, in recent years, metal microparticles and semiconductor microparticles with a very high level of dispersibility are desired as a carrier for use in a drug delivery system (DDS). For this purpose, a method for bonding PEG to microparticles is also known. For example, bonding PEG to metal microparticles or semiconductor microparticles through a thiol group is known (see patent document 4 (Japanese Patent Laid-Open No. 80903/2003) and patent document 5 (Japanese Patent Laid-Open No. 300253/2004)). Further, bonding PEG to metal microparticles, metal oxide microparticles, or semiconductor microparticles through a mercapto group or trifunctional silanol group is also known (patent document 6 (Japanese Patent Laid-Open No. 200050/2001)). These techniques, however, are not suitable for application to titanium oxide particles. The reason for this is that the thiol or mercapto group cannot be stably bound to titanium oxide and, further, the trifunctional silanol groups are mutually three-dimensionally condensation polymerized to produce a polymer which, in some cases, disadvantageously covers the surface of the titanium oxide particles resulting in lowered catalytic activity of titanium oxide.

Further, surface modified titanium oxide nanoparticles produced by bonding a hydrophilic polymer such as polyacrylic acid to titanium oxide nanoparticles by an ester bond through a carboxyl group are also known (see patent document 7 (WO 2004/087577)). This technique is intended to use an anionic polymer such as polyacrylic acid.

Further, there has also been proposed a technique in which ultrasonic waves of 35 to 42 kHz are applied to titanium oxide having a particle size of 2 to 3 mm to generate hydroxy radicals which decompose organic matter (see, for example, patent document 8 (Japanese Patent Laid-Open No. 26406/2003)).

Meanwhile, a technique in which an endiol ligand is bound to the surface of a metal oxide such as TiO₂ to vary optical properties of nano particles is known (see, for example, non-patent document 1 (T. Rajh, et al., J. Phys. Chem. B 2002, 106, 10543-10552)). This technique, however, is not a technique in which a polymer is bound to a metal oxide.

Patent document 1: Japanese Patent Laid-Open No. 307524/1990
Patent document 2: Japanese Patent Laid-Open No. 60651/2002
Patent document 3: WO 2005/120590 A1
Patent document 4: Japanese Patent Laid-Open No. 80903/2003
Patent document 5: Japanese Patent Laid-Open No. 300253/2004
Patent document 6: Japanese Patent Laid-Open No. 200050/2001
Patent document 7: WO 2004/087577
Patent document 8: Japanese Patent Laid-Open No. 26406/2003

Non-patent document 1: T. Rajh, et al., J. Phys. Chem. B 2002, 106, 10543-10552

### SUMMARY OF THE INVENTION

The present inventors have now found that bonding a nonionic hydrophilic polymer onto the surface of titanium oxide particles through at least one functional group selected from carboxyl group, amino group, diol group, salicylic acid group, and phosphoric acid group can improve retentivity in blood and accumulation in cancer cells while satisfactorily developing the catalytic activity of titanium oxide particles to be excited upon exposure to ultrasonic waves or ultraviolet light.

Accordingly, the object of the present invention is to provide titanium oxide composite particles can improve retentivity in blood and accumulation in cancer cells while satisfactorily developing the catalytic activity of titanium oxide particles to be excited upon exposure to ultrasonic waves or ultraviolet light, and to provide a dispersion thereof. Specifically, according to the titanium oxide composite particles of the present invention, in the case where the object to be killed is cancer cells, the effect of treating cancer by ultrasonic or ultraviolet light irradiation can be significantly improved. Therefore, the titanium oxide composite particles according to the present invention can also be utilized as an ultrasonic cancer treatment enhancer for enhancing ultrasonic cancer treatment which is carried out by applying ultrasonic waves to an affected part.

According to the present invention, there is provided titanium oxide composite particles comprising:
titanium oxide particles; and
a nonionic hydrophilic polymer bound to the surface of the titanium oxide particles through at least one functional group selected from carboxyl group, amino group, diol group, salicylic acid group, and phosphoric acid group, wherein the nonionic hydrophilic polymer is polyethylene glycol, and the bonding amount of the hydrophilic polymer per unit weight of the titanium oxide composite particles is 0.3 to 0.5 g/g.

There is also provided a dispersion liquid comprising the titanium oxide composite particles and a solvent for dispersing the particles.

There is provided a process for producing titanium oxide composite particles according to the first aspect, the process comprising:
dispersing titanium oxide particles and polyethylene glycol modified with at least one functional group selected from carboxyl group, amino group, diol group, salicylic acid group, and phosphoric acid group, in an aprotic solvent, to obtain a dispersion liquid; and
heating the dispersion liquid at 80 to 220°C to obtain a titanium oxide composite particles.

There is provided a process for producing titanium oxide composite particles according to the second aspect, the process comprising:
dispersing titanium oxide particles, a ligand molecule containing at least one functional group selected from diol group, salicylic acid group and phosphoric acid group, and , polyethylene glycol in an aprotic solvent, to obtain a dispersion liquid; and
heating the dispersion liquid at 80 to 220°C to obtain titanium oxide composite particles.

There is provided a process for producing titanium oxide composite particles according to the third aspect, the process comprising:
dispersing titanium oxide particles and a polycarboxylic acid in an aprotic solvent to obtain a dispersion liquid;
heating the dispersion liquid at 80 to 220°C to obtain a dispersion liquid of the titanium oxide particles to which the polycarboxylic acid is bound; and
adding polyethylene glycol modified with the functional group to the dispersion liquid thus heated to allow a reaction to proceed in an aqueous solution at pH 8 to 10 to obtain the titanium oxide composite particles.

There is provided a process for producing titanium oxide composite particles according to the fourth aspect, the process comprising:
dispersing titanium oxide particles and a polyamine in an aprotic solvent to obtain a dispersion liquid;
heating the dispersion liquid at 80 to 220°C to obtain a dispersion liquid of titanium oxide particles to which the polyamine is bound; and
adding polyethylene glycol modified with the functional group to the dispersion liquid thus heated to allow a reaction to proceed in an aqueous solution at pH 8 to 10 to obtain the titanium oxide composite particles.

There is provided a process for producing titanium oxide composite particles according to the fifth aspect, the process comprising:
dispersing titanium oxide particles and a ligand molecule containing at least one functional group selected from diol group, salicylic acid group, and phosphoric acid group in an aprotic solvent to obtain a dispersion liquid;
heating the dispersion liquid at 80 to 220°C to obtain a dispersion liquid of titanium oxide particles to which the ligand molecule is bound; and
adding polyethylene glycol to the dispersion liquid thus heated to obtain the titanium oxide composite particles.

### BRIEF DESCRIPTION OF THE DRAWINGS

[Fig. 1] Fig. 1 is a diagram showing an example of the titanium oxide composite particle according to the present invention wherein numeral 1 represents a titanium oxide particle and numeral 2 represents polyethylene glycol.
[Fig. 2] Fig. 2 is a diagram showing the decomposition rates (%) of methylene blue as measured in Example 4 using each TiO₂/PEG dispersion liquid prepared in Examples 1 to 3.
[Fig. 3] Fig. 3 is a diagram showing the relationship between the average particle diameter of TiO₂/PEG prepared in Example 1 and the concentration of sodium chloride in the dispersion liquid, as measured in Example 5.
[Fig. 4] Fig. 4 is a diagram showing the relationship between the average particle diameter of TiO₂/PEG prepared in Example 1 and the pH of the dispersion liquid, as measured in Example 6.
[Fig. 5] Fig. 5 is a diagram showing the average particle diameter of TiO₂/PEG and titanium oxide particles after standing at room temperature for one hour and for 24 hours, as measured in Example 7.
[Fig. 6] Fig. 6 is a diagram showing an image of the titanium oxide nanoparticle dispersion liquid and the TiO₂/PEG-containing dispersion liquid prepared in Example 1, after treatment with a 10 mM phosphate buffer solution containing 0.1 M sodium chloride and standing at room temperature for one hour, as photographed in Example 8, wherein a petri dish containing the titanium oxide nanoparticle dispersion liquid is shown on the right side of the drawing and a petri dish containing the TiO₂/PEG-containing dispersion liquid is shown on the left side of the drawing.
[Fig. 7] Fig. 7 is a diagram showing the relationship between the concentration of TiO₂/PEG and the cell survival rate (%), as measured in Example 9 using the TiO₂/PEG dispersion liquid prepared in Example 1.
[Fig. 8] Fig. 8 is a diagram showing the cell killing rates (%), as measured in Example 10 using or not using TiO₂/PEG prepared in Example 1.
[Fig. 9] Fig. 9 is a diagram showing the relationship between the average particle diameter of the polyethylene glycol-bound titanium dioxide nanoparticles prepared in Example 13 and the concentration of sodium chloride in the dispersion liquid, as measured in Example 21.
[Fig. 10] Fig. 10 is a diagram showing the relationship between the average particle diameter of the polyethylene glycol-bound titanium dioxide nanoparticles prepared in Example 15 and the pH of the dispersion liquid, as measured in Example 22.
[Fig. 11] Fig. 11 is a diagram showing the relationship between the average particle diameter of the polyethylene glycol-bound titanium dioxide nanoparticles prepared in Example 15 and the standing time, as measured in Example 23.
[Fig. 12] Fig. 12 is a diagram showing the decomposition rates of methylene blue, i.e., the reductions in absorbance, as measured in Example 24 in which each polyethylene glycol-bound titanium dioxide nanoparticle solution prepared in Examples 13 to 15 was irradiated with ultraviolet light with a wavelength of 340 nm at an exposure of 5 J/cm².

### DETAILED DESCRIPTION OF THE INVENTION

### Titanium oxide composite particles and dispersion thereof

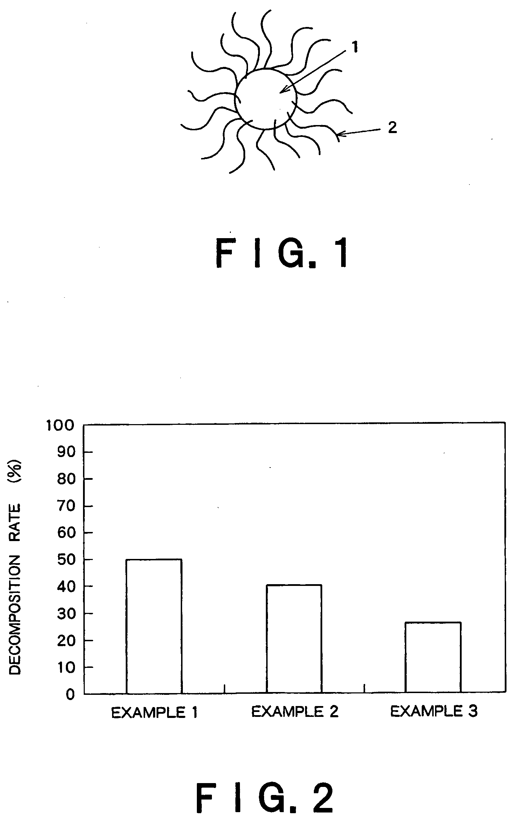

The titanium oxide composite particles according to the present invention comprises titanium oxide particles and polyethylene glycol. Fig. 1 shows an example of the titanium oxide composite particles. As shown in Fig. 1, the titanium oxide composite particle is the one in which the polyethylene glycol 2 is bonded to the surface of the titanium oxide particle 1. The bonding between the titanium oxide particle 1 and the hydrophilic polymer 2 is formed through at least one functional group selected from carboxyl, amino, diol, salicylic acid, and phosphoric acid groups. Specifically, since these functional groups form a strong bond to titanium oxide, the bonding of the polyethylene glycol can be retained despite a high catalytic activity of the titanium oxide particles. In the present invention, the bonding form may be on such a level that can ensure dispersibility 24 to 72 hr after administration into the body from the viewpoint of ensuring retention in blood. The bonding form, however, is preferably a covalent bond because of its excellent dispersion stability under physiological conditions and freedom from polymer liberation and no significant damage to normal cells upon ultrasonic irradiation or ultraviolet irradiation.

It is considered that carboxyl, amino, diol, salicylic acid and phosphoric acid groups can ensure a large portion of naked parts on the surface of the titanium oxide particles as shown in Fig. 1, unlike functional groups such as trifunctional silanol groups which condensation-polymerize to each other three-dimensionally to disadvantageously cover the surface of the titanium oxide particles with the resultant polymer. As a result, the catalytic activity of the titanium oxide particles can be satisfactorily developed while suppressing the deactivation of the titanium oxide particles caused by the covering of the surface with the polymer.

The titanium oxide composite particles can be dispersed on a high level by hydration without charging, even in an aqueous solvent around a neutral state, in which the dispersion of titanium oxide particles are considered difficult, since the polyethylene glycol bonded to the surface of the titanium oxide particles is nonionic. Also, retention in blood can be ensured on a level high enough to realize arrival at the target site (tumor), since the polyethylene glycol is uncharged so that blood protein can be less likely to be electrostatically adsorbed, making it easier to avoid incorporation e.g. into a reticuloenodothelial system, renal excretion or, liver incorporation. Further, with the use of the uncharged hydrophilic polymer, i.e. the polyethylene glycol the polymer can easily arrive at the surface of cancer cells at high density, contributing to excellent accumulation in the cancer cells. Accordingly, the titanium oxide composite particles according to the present invention can be delivered within the body to be efficiently accumulated in the cancer cells, while retaining a high level of dispersibility and a high level of retention in blood. The titanium oxide composite particles according to the present invention is therefore suitable for systemic administration e.g. through instillation and is particularly suitable for the treatment of a wide variety of cancers from a surface part to a deep part.

In a preferred embodiment of the present invention, the functional group is a diol group, more preferably an enediol group, still more preferably an α-diol group. With the use of these functional groups, a high level of bonding of polyethylene glycol to the titanium oxide particles can be realized.

In a more preferred embodiment of the present invention, the diol, salicylic acid, or phosphoric acid groups are provided by ligand molecules having these functional groups, and the polyethylene glycol is bound onto the surface of the titanium oxide particles through the ligand molecule. Preferred ligand molecules are cyclic molecules, which can further improve the strength of the bonding of the polyethylene glycol to the titanium oxide particles.

In a preferred embodiment of the present invention, the ligand molecule further comprises at least one functional group selected from carboxyl and amino groups bound to the polyethylene glycol. In this embodiment, at least one functional group selected from diol, salicylic acid and phosphoric acid groups can realize strong bond to titanium oxide particles and, at the same time, can realize strong bonding between the carboxyl group and/or amino group and the polyethylene glycol. As a result, particularly excellent bonding of the polyethylene glycol to the titanium oxide particles can be realized.

Preferred examples of the diol group-containing ligand molecules include protocatechic acid, gallic acid, methyldopa, quinic acid, and the combinations thereof from the viewpoints of hydrosolubility and bonding to titanium dioxide. Other examples of the ligand molecules include caffeic acid, 3,4-dihydrobenzaldehyde, 3,4-dihydrobenzoic acid ethyl ester, 3,4-dihydroxybenzyl alcohol, 3,4-dihydroxy-3-cyclobutene-1,2-dione, dl-3,4-dihydroxymandelic acid, 3-methoxycatechol, 2-dihydroxynaphthalene, dl-3-(3,4-dihydroxyphenyl)alanine, 2-(3,4-dihydroxyphenyl)ethyl alcohol, 2,3-dihydroxypyridine, and 2,3-dihydroxyquinozaline.

Preferred examples of the salicylic acid group-containing ligand molecules include 4-aminosalicylic acid from the viewpoints of hydrosolubility and bonding to titanium dioxide.

Preferred examples of the phosphoric acid group-containing ligand molecules include aminomethylphosphonic acid, phosphonocarboxylic acid, and 3-phosphonoalanine from the viewpoints of hydrosolubility and bonding to titanium dioxide. Other examples of the phosphoric acid group-containing ligand molecules include 1-aminopropylphosphonic acid, 3-aminopropylphosphonic acid, 1-aminoethylphosphonic acid, 2-aminoethylphosphonic acid, 3-phosphonopropionic acid, 2-aminoethyl dihydrogen phosphate, 2-hydroxy-3-oxopropyl dihydrogen phosphate, o-phosphonoserine, and 2-phosphoglyceric acid.

The hydrophilic polymer used in the present invention is polyethylene glycol (PEG), Prefered degree of polymerization of the hydrophilic polymer is 34 to 500, more preferably 34 to 50.

In a preferred embodiment of the present invention, it is preferred to use polyethylene glycol as the hydrophilic polymer and to use a carboxyl group as the functional group. In this embodiment, the molecular weight ratio between polyethylene glycol and the carboxyl group is preferably 15000:20 to 400000:20, more preferably 15000:20 to 40000:20. The molecular weight of polyethylene glycol is preferably 1500 to 40000, more preferably 1500 to 4000.

In a preferred embodiment of the present invention, the functional group of the carboxyl and/or amino groups is provided by carboxylic acid and/or amine. In this case, at least the end of the PEG is preferably modified with the carboxylic acid or amine. More preferably, the carboxylic acid or amine and the hydrophilic polymer get together to form a copolymer. Thus, the titanium oxide particles and the PEG can be bonded strongly. Specifically, a copolymer of a carboxylic acid or amine and a PEG can be used as the functional group-modified nonionic hydrophilic polymer. These copolymers strongly bond as a linker on the surface of the titanium oxide particles, and are able to have the residue of the functional group, which is not participating in bonding to titanium oxide, bonded by functional substances such as fluorescent dyes or biopolymers, since the number of carboxyl groups and amino groups can be increased. Preferred examples of such copolymers include maleic acid-polyethylene glycol copolymers.

In another preferred embodiment of the present invention, the functional group of carboxyl group and/or amino group is provided by a polycarboxylic acid or a polyamine as the linker. These polymeric compounds strongly bond as a linker on the surface of the titanium oxide particles, and are able to have the residue of the functional group, which is not participating in bonding to titanium oxide, bonded by functional substances such as fluorescent dyes or biopolymers, since the number of carboxyl groups and amino groups can be increased. Preferred examples of the polycarboxylic acids include polyacrylic acids, polymaleic acids, acrylic acid-maleic acid copolymers, and acrylic acid-sulfonic acid copolymers. Preferred examples of the polyamines include polyethyleneimines, polyvinylamines, and polyallylamines. Further, it is possible to use compounds containing both carboxyl and amino groups, and preferred examples of such compounds include polyamino acids such as polyornithine and polylysine.

In a preferred embodiment of the present invention, the composite particles further comprise a compound other than the polycarboxylic acid or polyamine, which has a functional group capable of forming a chemical bond together with at least one functional group selected from carboxyl and amino groups, as a second linker for bonding a linker formed of a polycarboxylic acid or a polyamine to a hydrophilic polymer. Specifically, it is possible to bond the second linker to a linker formed of a polycarboxylic acid or a polyamine, having the second linker bonded to by a hydrophilic polymer. For example, heterobifunctional crosslinkers used in bonding tissue-derived molecules to each other through different functional groups are considered as the second linker. Specific examples of the second linker include N-hydroxysuccinimide, N-[α-maleimidoacetoxy]succinimide esters, N-[β-maleimidopropyloxy]succinimide esters, N-β-maleimidopropionic acid, N-[β-maleimidopropionic acid]hydrazide TFA, 1-ethyl-3-[3-dimethylaminopropyl]carbodiimide hydrochloride, N-ε-maleimidocaproic acid, N-[ε-maleimidocaproic acid]hydrazide, N-[ε-maleimidocaproyloxy]succinimide esters, N-[γ-maleimidobutyryloxy]succinimide esters, N-κ-maleimidoundecanic acid, N-[κ-maleimidoundecanic acid]hydrazide, succinimidyl 4-[N-maleimidomethyl]-cyclohexane-1-carboxy-[6-amidocaproate], succinimidyl 6-[3-(2-pyridyldithio)-propionamide]hexanoate, m-maleimidobenzoyl-N-hydroxysuccinimide esters, 4-[4-N-maleimidophenyl]butyric acid hydrazide HCL, 3-[2-pyridyldithio]propionylhydrazide, N-[p-maleimidophenyl] isocyanate, N-succinimidyl [4-azidophenyl]-1,3'-dithiopropionate, N-succinimidyl S-acetylthioacetate, N-succinimidyl S-acetylthiopropionate, succinimidyl 3-[bromoacetamido]propionate, N-succinimidyl iodoacetate, N-succinimidyl[4-iodoacetyl]aminobenzoate, succinimidyl 4-[N-maleimidomethyl]-cyclohexane-1-carboxylate, succinimidyl 4-[p-maleimidophenyl]butyrate, succinimidyl 6-[β-maleimidopropionamide)hexanonate, 4-succinimidyloxycarbonyl-methyl-α[2-pyridyldithio]toluene, N-succinimidyl 3-[2-pyridyldithio]propionate, N-[ε-maleimidocaproyloxy]sulfosuccinimide esters, N-[γ-maleimidobutyryloxy]sulfosuccinimide esters, N-[κ-maleimidoundecanoyloxy]sulfosuccinimide esters, sulfosuccinimidyl-6-[α-methyl-α-(2-pyridyldithio)toluamido]hexanonate, sulfosuccinimidyl 6-[3'-(2-pyridyldithio)propionamido]hexanonate, m-maleimidobenzoyl-N-hydroxysulfosuccinimide ester, sulfosuccinimidyl [4-iodoacetyl]aminobenzoate, sulfosuccinimidyl 4-N-maleimidomethyl]-cyclohexane-1-carboxylate, sulfosuccinimidyl 4-[p-maleimidophenyl]-butyrate, and N-[ε-trifluoroacetylcaproyloxy]succinimide esters. Further, the second linker may be formed of a plurality of types of linkers which enables other linkers to be bound to each other.

In a preferred embodiment of the present invention, a functional group other than the carboxyl and amino groups, as a functional group capable of bonding to the linker or second linker, may be bonded to the hydrophilic polymer to ensure strong bonding to the linker. Such functional groups other than the carboxyl and amino groups include carbohydrate, sulfide, succinimide, maleimide, carbodiimide, and hydrazide groups.

In a preferred embodiment of the present invention, a biopolymer may be bonded to the residue of the carboxyl and/or amino groups, which is not participating in bonding to titanium oxide. For example, the capability of targetting to cancer cells can be further enhanced by imparting a biochip, such as an antibody, to the titanium oxide composite particles.

In a preferred embodiment of the present invention, the composite particles further comprise a compound other than polyols, polyphosphoric acids, polycarboxylic acids, and polyamines, which has a functional group which gets together with at least one functional group selected from carboxyl, amino, diol, salicylic acid, and phosphoric acid groups to form a chemical bond, as a second linker for bonding the ligand molecule to the hydrophilic polymer. Specifically, it is possible to bond the second linker to a diol, salicylic acid, phosphoric acid, carboxyl or amino group (hereinafter often referred to as first linker), which may be contained in the ligand molecule, having the second linker bonded to by a hydrophilic polymer. For example, heterobifunctional crosslinkers used in bonding tissue-derived molecules to each other through different functional groups are considered as the second linker. Specific examples of the second linker include N-hydroxysuccinimide, N-[a-maleimidoacetoxy]succinimide esters, N-[β-maleimidopropyloxy]succinimide esters, N-[β-maleimidopropionic acid, N-[β-maleimidopropionic acid]hydrazide TFA, 1-ethyl-3-[3-dimethylaminopropyl]carbodiimide hydrochloride, N-ε-maleimidocaproic acid, N-[ε-maleimidocaproic acid]hydrazide, N-[ε-maleimidocaproyloxy]succinimide esters, N-[γ-maleimidobutyryloxy]succinimide esters, N-κ-maleimidoundecanic acid, N-[κ-maleimidoundecanic acid]hydrazide, succinimidyl 4-[N-maleimidomethyl]-cyclohexane-1-carboxy-[6-amidocaproate], succinimidyl 6-[3-(2-pyridyldithio)-propionamide]hexanoate, m-maleimidobenzoyl N-hydroxysuccinimide esters, 4-[4-N-maleimidophenyl]butyric acid hydrazide HCL, 3-[2-pyridyldithio]propionylhydrazide, N-[p-maleimidophenyl] isocyanate, N-succinimidyl [4-azidophenyl]-1,3'-dithiopropionate, N-succinimidyl S-acetylthioacetate, N-succinimidyl S-acetylthiopropionate, succinimidyl 3-[bromoacetamido]propionate, N-succinimidyl iodoacetate, N-succinimidyl[4-iodoacetyl]aminobenzoate, succinimidyl 4-[N-maleimidomethyl]-cyclohexane-1-carboxylate, succinimidyl 4-[p-maleimidophenyl]butyrate, succinimidyl 6-[β-maleimidopropionamide)hexanonate, 4-succinimidyloxycarbonyl-methyl-α[2-pyridyldithio]toluene, N-succinimidyl 3-[2-pyridyldithio]propionate, N-[ε-maleimidocaproyloxy]sulfosuccinimide esters, N-[γ-maleimidobutyryloxy]sulfosuccinimide esters, N-[κ-maleimidoundecanoyloxy]sulfosuccinimide esters, sulfosuccinimidyl-6-[α-methyl-α-(2-pyridyldithio)toluamido]hexanonate, sulfosuccinimidyl 6-[3'-(2-pyridyldithio)propionamido]hexanonate, m-maleimidobenzoyl-N-hydroxysulfosuccinimide ester, sulfosuccinimidyl [4-iodoacetyl]aminobenzoate, sulfosuccinimidyl 4-N-maleimidomethyl]-cyclohexane-1-carboxylate, sulfosuccinimidyl 4-[p-maleimidophenyl]-butyrate, and N-[ε- trifluoroacetylcaproyloxy]succinimide esters. Further, the second linker may be formed of a plurality of types of linkers which enables other linkers to be bound to each other.

In a preferred embodiment of the present invention, a functional group other than the diol, salicylic acid, and phosphoric acid groups, as a functional group capable of bonding to the first or second linker, may be bonded to the PEG to ensure strong bonding to the linker. Examples of such other functional groups include carbohydrate, sulfide, succinimide, maleimide, carbodiimide, and hydrazide groups.

In a preferred embodiment of the present invention, a hydrophilic polymer may be bonded to the residue of the functional group of the ligand molecule, which is not participating in bonding to titanium oxide. The bonding form is not particularly limited. Preferred examples of the bonding to the residue include bonding by graft polymerization using azo group-containing polyethylene glycol and a carboxyl group of the ligand molecule, and besides bonding by graft polymerization to a phenolic hydroxyl group, a vinyl group, or an aromatic ring. Preferred examples of another bonding to the residue include bonding using succinimide group-containing polyethylene glycol and amino group of the ligand molecule, and besides bonding using reactive functional groups such as carbohydrate, sulfide, succinimide, maleimide, carbodiimide, isocyanate isothiocyanate, and hydrazide groups. The ligand molecule and the hydrophilic polymer may also be bonded to each other indirectly through a second linker.

In a preferred embodiment of the present invention, a fluorescent dye or a biopolymer is bonded to the residue of the PEG, which is not participating in bonding to the ligand molecule. For example, the capability of targetting to cancer cells can be further enhanced by imparting a biochip such as an antibody or the like to the titanium oxide composite particles. The bonding form of the biopolymer is not particularly limited. Preferred examples of the bonding to the residue include bonding by graft polymerization using azo group-containing polyethylene glycol and a carboxyl group of the ligand molecule, and besides bonding by graft polymerization to a phenolic hydroxyl group, a vinyl group, or an aromatic ring. Preferred examples of another bonding to the residue include bonding using succinimide group-containing polyethylene glycol and amino group of the ligand molecule, and besides bonding using reactive functional groups such as carbohydrate, sulfide, succinimide, maleimide, carbodiimide, isocyanate, isothiocyanate, and hydrazide groups. The ligand molecule and the hydrophilic polymer may also be bonded to each other indirectly through a second linker.

In a preferred embodiment of the present invention, the titanium oxide particles are preferably anatase-type titanium oxide or rutile-type titanium oxide. In the case of using catalytic activity by ultraviolet light or ultrasonic irradiation, anatase-type titanium oxide is preferred. On the other hand, in the case of using properties such as high refractive index as in cosmetic products, rutile-type titanium oxide is preferred.

In a preferred embodiment of the present invention, the titanium oxide composite particles used in the present invention have a particle diameter of 20 to 200 nm, more preferably 50 to 200 nm, still more preferably 50 to 150 nm. Within this particle diameter range, upon administration into the body of a patient aiming at the arrival at tumor, as in the drug delivery system, the titanium oxide composite particles efficiently arrive at and are efficiently accumulated in cancer tissues by EPR effect. As described above, upon exposure to ultrasonic waves at 400 kHz to 20 MHz or ultraviolet light, specific generation of radical species takes place. Accordingly, the cancer tissue can be killed with high efficiency by ultrasonic or ultraviolet irradiation.

In another preferred embodiment of the present invention, when the titanium oxide composite particles have a particle diameter of less than 50 nm (for example, a few nanometers), the EPR effect can also be attained by increasing the apparent size. Specifically, when the titanium oxide composite particles are bound, for example, by a method in which semiconductor particles are linked to each other through a polyfunctional linker so as to take a secondary particle form having a particle diameter of 50 to 150 nm, a high cancer treatment effect can be realized by EPR effect. In still another preferred embodiment of the present invention, in order to utilize the EPR effect, titanium oxide composite particles can also be included in an inclusion of a medicament such as liposome.

In the present invention, the particle diameter of the semiconductor particles can be measured by a dynamic light scattering method. Specifically, the particle diameter of the semiconductor particles may be obtained as a value expressed in terms of Z-average size provided by a cumulant analysis with a particle size distribution measuring apparatus (Zetasizer Nano, manufactured by Malvern Instruments Ltd.).

In a preferred embodiment of the present invention, the titanium oxide composite particles have a zeta potential of -20 to +20 mV, more preferably -10 to +10 mV, still more preferably -5 to +5 mV, most preferably -3 to +3 mV. Within this range, the titanium oxide composite particles are substantially uncharged as a whole. Accordingly, the effect of improving retention in blood and accumulation in cancer cells can be maximized by using PEG.

In a preferred embodiment of the present invention, the number of moles of the carboxyl or amino group per unit weight of the titanium oxide composite is 1 × 10⁻⁹ to 1 × 10⁻⁴ mol/g, more preferably 1 × 10⁻⁹ to 1 × 10⁻⁶ mol/g. Within this range, the retention in blood and accumulation of the titanium oxide composite particles in cancer cells can be improved while satisfactorily developing the catalytic activity of the titanium oxide composite particles.

According to the present invention, the bonding amount of the hydrophilic polymer per unit weight of the titanium oxide composite particles is 0.3 to 0.5 g/g from the viewpoint of dispersibility. Within this range, the retention in blood and accumulation of the titanium oxide composite particles into cancer cells can be improved while satisfactorily developing the catalytic activity of the titanium oxide composite particles.

The titanium oxide composite particles usable in the present invention include not only a single type of titanium oxide composite particles but also a mixture or composite of a plurality of types of semiconductor particles. Specific examples thereof include a composite of a titanium oxide composite particle and an iron oxide nanoparticle, a composite of a titanium oxide composite particle and platinum, and a silica-coated titanium oxide.

In a preferred embodiment of the present invention, the titanium oxide composite particles are in the form of a dispersion liquid in which the titanium oxide composite particles are dispersed in a solvent. In the dispersion liquid form, the titanium oxide composite particles can be efficiently administered into the body of the patient by various methods such as instillation, injection, or coating. The liquidity of the dispersion liquid is not limited, and a high level of dispersibility can be realized over a wide pH range of 3 to 10. From the viewpoint of safety in administration into the body, preferably, the dispersion liquid has a pH value of 5 to 9, more preferably 5 to 8, particularly preferably neutral liquidity. In a preferred embodiment of the present invention, the solvent is an aqueous solvent, more preferably a pH buffer solution or physiological saline. The concentration of salt in the aqueous solvent is preferably 2 M or less, more preferably 200 mM or less from the viewpoint of safety in administration into the body. The content of the titanium oxide composite particles in the dispersion is preferably 0.001 to 1% by mass, more preferably 0.001 to 0.1% by mass. Within this range, the titanium oxide composite particles can be effectively accumulated in an affected part (tumor) in 24 to 72 hr after the administration. Specifically, the particles are likely to be accumulated in a high concentration in the affected part (tumor), and, at the same time, the dispersibility of the particles in the blood can be ensured and, consequently, aggregates are less likely to be formed, leading to an advantage that no secondary negative effect such as obstruction of blood after the administration does takes place.

The titanium oxide composite particles according to the present invention can be administered into the body of a patient by various methods such as instillation, injection, or coating. The use of the titanium oxide composite particles through an administration route of intravenous or subcutaneous administration is particularly preferred from the viewpoint of reducing patient's burden by the so-called "DDS-like treatment" utilizing EPR effect attained by taking advantage of particle size and retention in blood. The titanium oxide composite particles administered into the body arrive at and are accumulated in cancer tissues as in the drug delivery system.

The titanium oxide composite particles according to the present invention can be rendered cytotoxic upon exposure to ultrasonic waves or ultraviolet light. The titanium oxide composite particles are administered into the body, undergo ultrasonic irradiation, and can be rendered cytotoxic upon the ultrasonic irradiation to kill cells. However, it should be noted that cells to be killed can be killed in vivo, as well as in vitro. In the present invention, the object to be killed is not particularly limited. However, cancer cells are preferred. Specifically, the titanium oxide composite particles according to the present invention can be activated upon ultrasonic or ultraviolet irradiation to kill cancer cells. Further, since the titanium oxide composite particles are not a photosensitizer such as fullerene or pigment, a problem with hypersensitivity to light at the stage of treatment after administration to a patient does not take place and a high level of safety can be realized.

In a preferred embodiment of the present invention, the cancer tissue in which titanium oxide composite particles have been accumulated is ultrasonicated. The frequency of ultrasonic waves used is preferably 400 kHz to 20 MHz, more preferably 600 kHz to 10 MHz, still more preferably 1 MHz to 10 MHz. The ultrasonic irradiation time should be properly determined by taking into consideration the position and size of the cancer tissue as the treatment object and is not particularly limited. Thus, the cancer tissue in the patient can be killed by ultrasonic irradiation with high efficiency to realize a high cancer treatment effect. The ultrasonic waves can be allowed to externally arrive at a deep part in the living body. When the ultrasonic waves are used in combination with the titanium oxide composite particles according to the present invention, the treatment of an affected part or target site, which is present in a deep part in the living body, in a noninvasive state, can be realized. Further, since the titanium oxide composite particles according to the present invention are accumulated in the affected part or target site, even ultrasonic waves having a very low intensity that does not adversely affect peripheral normal cells, can be allowed to act locally on only the site where the titanium oxide composite particles are accumulated.

In the meantime, the effect of killing cells through the activation of semiconductor particles upon ultrasonic irradiation can be attained by the generation of radical species upon ultrasonic irradiation. Specifically, the biological killing effect provided by the semiconductor particles is considered attributable to a qualitative and quantitative increase in radical species. The reason for this is estimated as follows. However, it should be noted that the following reason is hypothetical and the present invention is not limited to the following description. Specifically, when ultrasonic irradiation is solely used, hydrogen peroxide and hydroxyl radicals are generated in the system. According to the finding of the present inventors, the generation of hydrogen peroxide and hydroxyl radicals are accelerated in the presence of semiconductor particles such as titanium oxide particles. Further, in the presence of semiconductor particles, particularly in the presence of titanium oxide, it seems that the generation of superoxide anions and singlet oxygen is accelerated. The specific generation of radical species, in the case of using nanoparticles of nanometer order, is considered as a phenomenon significantly observed at a frequency of ultrasonic irradiation in the range of 400 kHz to 20 MHz, preferably in the range of 600 kHz to 10 MHz, more preferably in the range of 1 MHz to 10 MHz.

### Production process

According to a production process in the first embodiment of the present invention, the titanium oxide composite particles according to the present invention can be produced by bonding a nonionic hydrophilic polymer, i.e. PEG, modified with at least one functional group, selected from carboxyl, amino, diol, salicylic acid and phosphoric acid groups, to titanium oxide particles. The production of the titanium oxide composite particles by this process can be carried out, for example, by dispersing titanium oxide particles and PEG modified with at least one functional group, selected from carboxyl, amino, diol, salicylic acid and phosphoric acid groups, in an aprotic solvent, and heating the resultant dispersion liquid at 80 to 220°C, for example, for 1 to 16 hours. In a more preferred embodiment of the present invention, a copolymer of a carboxylic acid or an amine with a PEG is used as the nonionic hydrophilic polymer modified with a functional group. A maleic acid-polyethylene glycol copolymer is more preferred.

According to a production process in the second embodiment of the present invention, the titanium oxide composite particles according to the present invention can be produced by simultaneously dispersing titanium oxide particles, a ligand molecule, and a nonionic hydrophilic polymer, i.e. PEG, to bond them to each other. The production of the titanium oxide composite particles by this process can be carried out, for example, by dispersing titanium oxide particles, a ligand molecule containing at least one functional group selected from diol, salicylic acid and phosphoric acid groups, and PEG, in an aprotic solvent, and heating the resultant dispersion liquid at 80 to 220°C, for example, for 1 to 16 hr, to prepare titanium oxide composite particles.

According to a production process in the third embodiment of the present invention, the titanium oxide composite particles according to the present invention can be produced by first modifying titanium oxide particles with a carboxyl group-containing linker and then bonding a hydrophilic polymer, i.e. PEG, to the residue of the linker bound to the titanium oxide particles. The production of the titanium oxide composite particles by this process can be carried out, for example, by dispersing titanium oxide particles and a polycarboxylic acid in an aprotic solvent, heating the resultant dispersion liquid at 80 to 220°C to prepare a dispersion liquid of titanium oxide particles to which the polycarboxylic acid is bonded, and adding the functional group-modified PEG to the dispersion liquid to allow a reaction to proceed in an aqueous solution at pH 8 to 10 to prepare the titanium oxide composite particles. In a more preferred embodiment of the present invention, the polycarboxylic acid is polyacrylic acid, polymaleic acid, acrylic acid-maleic acid copolymers, or acrylic acid-sulfonic acid copolymer, more preferably polyacrylic acid. Thus, the linker can be strongly bound to the surface of the titanium oxide particles. Further, the functional group in the PEG modified with the functional group is preferably an amino group.

According to a production process in the fourth embodiment of the present invention, the titanium oxide composite particles according to the present invention can be produced by first modifying titanium oxide particles with an amino group-containing linker and then bonding a hydrophilic polymer, i.e. PEG, to the residue of the linker bound to the titanium oxide particles. The production of the titanium oxide composite particles by this process can be carried out, for example, by dispersing titanium oxide particles and a polyamine in an aprotic solvent, heating the resultant dispersion liquid at 80 to 220°C to prepare a dispersion liquid of titanium oxide particles to which the polyamine has been bound, and adding the functional group-modified PEG to the dispersion liquid to allow a reaction to proceed in an aqueous solution at pH 8 to 10 to prepare the titanium oxide composite particles. In a more preferred embodiment of the present invention, the polyamine is polyethyleneimine, polyvinylamine, or polyallylamine, more preferably polyethyleneimine. According to this constitution, the linker can be strongly bound to the surface of the titanium oxide particles. Further, the functional group in the PEG modified with the functional group is preferably a succinimide group.

According to a production process in the fifth embodiment of the present invention, the titanium oxide composite particles according to the present invention can be produced by first modifying titanium oxide particles with a ligand molecule and then bonding a hydrophilic polymer, i.e. PEG, to the residue of the ligand molecule bound to the titanium oxide particle. The production of the titanium oxide composite particles by this process can be carried out, for example, by dispersing titanium oxide particles and a ligand molecule containing at least one functional group selected from diol, salicylic acid, and phosphoric acid groups in an aprotic solvent, heating the resultant dispersion liquid at 80 to 220°C, for example, for 1 to 16 hours to prepare a dispersion liquid of titanium oxide particles to which the ligand molecule is bonded, and adding PEG to the dispersion liquid to prepare the polyethylene glycol-bound titanium dioxide nanoparticle.

In each of the embodiments, an aprotic solvent is used as a solvent. This is because there is a possibility that the use of a protonic solvent results in difficulty in the realization of a high level of dispersion since the protonic solvent hinders a bonding reaction such as the formation of an ester bond by dehydration. Preferred examples of the aprotic solvent include dimethylformamide, dioxane, and dimethylsulfoxide.

In a preferred embodiment of the present invention, it is possible to obtain a dispersion liquid of titanium oxide particles to which a polycarboxylic acid or a polyamine is bonded and then, before the addition of a nonionic hydrophilic polymer, i.e. PEG, to the dispersion liquid, to add a second linker for bonding the linker to the hydrophilic polymer to allow a reaction to proceed, having the second linker bond to the polyamine. This second linker is a compound other than a polycarboxylic acid or a polyamine, which contains a functional group capable of forming a chemical bond to at least one functional group selected from carboxyl and amino groups. Specific examples of such compounds are as described above.

In a preferred embodiment of the present invention, it is possible to obtain a dispersion liquid of titanium oxide particles, to which a ligand molecule is bonded and then, before the addition of a nonionic hydrophilic polymer, i.e. PEG, to the dispersion liquid, to add a second linker for bonding the linker to the hydrophilic polymer to allow a reaction to proceed, having the second linker bond to the polyamine. This second linker is a compound other than the polyol, polyphosphoric acid, polycarboxylic acid, and polyamine, which contains a functional group capable of forming a chemical bond to at least one functional group selected from diol, salicylic acid, phosphoric acid, carboxyl and amino groups. Specific examples of such compounds are as described above.

In a preferred embodiment of the present invention, in each of the above embodiments, the photocatalytic titanium oxide nanoparticles are purified by separating polyethylene glycol-bound titanium dioxide nanoparticles from the hydrophilic polymer remaining unbonded.

### EXAMPLES

### Example 1: Introduction of maleic acid-type polyethylene glycol into titanium oxide particles

Titanium tetraisopropoxide (3.6 g) was mixed with 3.6 g of isopropanol, and the mixture was added dropwise to 60 ml of ultrapure water under ice cooling for hydrolysis. After the dropwise addition, the mixture was stirred at room temperature for 30 min. After the stirring, 1 ml of 12 N nitric acid was added dropwise thereto, and the mixture was stirred at 80°C for 8 hr for peptization. After the completion of the peptization, the mixture was filtered through a 0.45-µm filter and was subjected to solution exchange with a desalination column (PD-10, manufactured by Amersham Bioscience) to prepare an acidic titanium oxide sol having a solid content of 1%. This titanium oxide sol was placed in a 100-ml vial bottle and was ultrasonicated at 200 kHz for 30 min in an ultrasonic generator MIDSONIC 200 (manufactured by KAIJO Corporation). The average dispersed particle diameter after the ultrasonication was measured by a dynamic light scattering method. This measurement was carried out by diluting the ultrasonicated titanium oxide sol with 12 N nitric acid by a factor of 1000, charging 0.1 ml of the dispersion liquid in a quartz measurement cell, setting various parameters of the solvent to the same values as water, and measuring the dispersed particle diameter at 25°C with Zetasizer Nano ZS (manufactured by SYSMEX CORPORATION). As a result, it was found that the dispersed particle diameter was 20.2 nm. The titanium oxide sol solution was concentrated at a temperature of 50°C using an evaporating dish to finally prepare an acidic titanium oxide sol having a solid content of 20%.

Next, 5 ml of water was added to 1 g of a copolymer of polyoxyethylene-monoallyl-monomethyl ether with maleic anhydride (average molecular weight; 33,659, manufactured by NOF Co., Ltd.). The mixture was hydrolyzed, and the hydrolyzate was then lyophilized. After the completion of the reaction, the lyophilized product was dissolved in 5 ml of a dimethylformamide (DMF) solution to prepare a 200 mg/ml polyethylene glycol solution. The polyethylene glycol solution (1.875 ml) thus obtained was added to 27.725 ml of a DMF solution, and 0.9 ml of the anatase-type titanium oxide sol prepared above was added thereto, followed by stirring for mixing. The solution was transferred to a hydrothermal reaction vessel (HU-50, manufactured by SAN-Al Science Co. Ltd.), a reaction was allowed to proceed at 150°C for 5 hr. After the completion of the reaction, the reaction vessel was cooled to a temperature of 50°C or below. DMF was removed by an evaporator, and 10 ml of distilled water was added to the residue to prepare a dispersion liquid of polyethylene glycol-bound titanium oxide nanoparticles (TiO₂/PEG). Further, the dispersion liquid was subjected to HPLC [AKTA Purifier (manufactured by Amersham Biosciences), column: HiPrep 16/60 Sephacryl S-300HR (manufactured by Amersham Biosciences), mobile phase: phosphate buffer solution (pH 7.4), flow rate: 0.3 ml/min]. As a result, an UV absorption peak was observed in a fraction passed through the column, and this fraction was recovered. The dispersion liquid was diluted with distilled water to prepare a 0.01% aqueous solution, and the dispersed particle diameter and zeta potential were measured by a dynamic light scattering method. This measurement was carried out with Zetasizer Nano ZS by charging 0.75 ml of a dispersion liquid of TiO₂/PEG into a zeta potential measuring cell, setting various parameters of the solvent to the same values as water, and conducting the measurement at 25°C. As a result of a cumulant analysis, it was found that the dispersed particle diameter and the zeta potential were 45.4 nm and 1.1 mV, respectively.

### Example 2: Introduction of polyethylene glycol into polyacrylic acid-bound titanium oxide nanoparticles

Titanium tetraisopropoxide (3.6 g) was mixed with 3.6 g of isopropanol, and the mixture was added dropwise to 60 ml of ultrapure water under ice cooling for hydrolysis. After the dropwise addition, the mixture was stirred at room temperature for 30 min. After the stirring, 1 ml of 12 N nitric acid was added dropwise thereto, and the mixture was stirred at 80°C for 8 hr for peptization. After the completion of the peptization, the mixture was filtered through a 0.45-µm filter and was subjected to solution exchange with a desalination column (PD-10, manufactured by Amersham Bioscience) to prepare an acidic titanium oxide sol having a solid content of 1 %. This titanium oxide sol was placed in a 100-ml vial bottle and was ultrasonicated at 200 kHz for 30 min. The average dispersed particle diameter after the ultrasonication was measured by a dynamic light scattering method. This measurement was carried out by diluting the ultrasonicated titanium oxide sol with 12 N nitric acid by a factor of 1000, charging 0.1 ml of the dispersion liquid in a quartz measurement cell, setting various parameters of the solvent to the same values as water, and measuring the dispersed particle diameter at 25°C with Zetasizer Nano ZS (manufactured by SYSMEX CORPORATION). As a result, it was found that the dispersed particle diameter was 20.2 nm. The titanium oxide sol solution was concentrated at a temperature of 50°C using an evaporating dish to finally prepare an acidic titanium oxide sol having a solid content of 20%

This acidic titanium oxide sol (0.6 ml) was dispersed in dimethylformamide (DMF) to give a total volume of 20 ml. DMF (10 ml) containing 0.3 g of polyacrylic acid (average molecular weight of 5000 (manufactured by Wako Pure Chemical Industries, Ltd.)) dissolved therein was added to the dispersion liquid, followed by stirring for mixing. The solution was transferred to a hydrothermal reaction vessel (HU-50 manufactured by SAN-Al science Co. Ltd.), and a reaction was allowed to proceed at 150°C for 5 hr. After the completion of the reaction, the reaction solution was cooled so that the temperature of the reaction vessel reached 50°C or below. Isopropanol in an amount of twice the amount of the reaction solution was added to the reaction solution. The mixture was allowed to stand at room temperature for 30 min and was then centrifuged under conditions of 2000 g and 15 min to recover precipitate. The surface of the recovered precipitate was washed with ethanol, and 1.5 ml of water was added thereto to give a dispersion liquid of polyacrylic acid-bound titanium oxide nanoparticles. This dispersion liquid was diluted with distilled water by a factor of 100, and the dispersed particle diameter and zeta potential were measured by a dynamic light scattering method. This measurement was carried out with Zetasizer Nano ZS by charging 0.75 ml of the dispersion liquid of the polyacrylic acid-bound titanium oxide nanoparticles in a zeta potential measuring cell, setting various parameters of the solvent to the same values as water, and conducting the measurement at 25°C. As a result, it was found that the dispersed particle diameter and the zeta potential were 53.6 nm and -45.08 mV, respectively.

250 µl of 0.8 M 1-ethyl-3-[3-dimethylaminopropyl] carbodiimide hydrochloride and 250 µl of N-hydroxysuccinimide were added to 5 ml of this dispersion liquid diluted with ultrapure water to give a concentration of 0.5 (w/v)%. The mixture was allowed to react with stirring at room temperature for one hr. The reaction solution was subjected to solution exchange by gel filtration with a desalination column (PD-10, manufactured by Amersham Pharmacia Bioscience) equilibrated with a 50 mM borate buffer solution (pH 9.0). Thereafter, a 50 mM borate buffer solution (pH 9.0) was added thereto to give a total volume of 9.5 ml. To this collected solution was added 2.5 ml of an aqueous solution of a polyethylene glycol derivative (SUNBRIGHT MEPA30-T (manufactured by NOF Co., Ltd.)) (regulated to 20 mg/ml). The mixture was gently stirred at room temperature for 6 hr. This solution was concentrated to a volume of 1 ml with VIVAPORE 7500 (manufactured by VIVASCIENCE). This concentrate was further subjected to HPLC [AKTA Purifier (manufactured by Amersham Biosciences), column: HiPrep 16/60 Sephacryl S-300HR, manufactured by Amersham Biosciences), mobile phase: phosphate buffer solution (pH 7.4), flow rate: 0.3 ml/min]. As a result, an UV absorption peak was observed in a fraction passed through the column, and this fraction was recovered. The dispersed particle diameter and zeta potential of the collected fraction were measured by a dynamic light scattering method. This measurement was carried out with Zetasizer Nano ZS by charging 0.75 ml of the collected fraction into a zeta potential measuring cell, setting various parameters of the solvent to the same values as water, and conducting the measurement at 25°C. As a result of a cumulant analysis, it was found that the dispersed particle diameter and the zeta potential were 80.7 nm and -6.329 mV, respectively. The above results show that the introduction of polyethylene glycol into the polyacrylic acid-bound titanium oxide nanoparticles (TiO₂/PEG) reduced the surface charge.

### Example 3: Introduction of polyethylene glycol into polyethyleneimine-bound titanium oxide nanoparticles

Titanium tetraisopropoxide (3.6 g) was mixed with 3.6 g of isopropanol, and the mixture was added dropwise to 60 ml of ultrapure water under ice cooling for hydrolysis. After the dropwise addition, the mixture was stirred at room temperature for 30 min. After the stirring, 1 ml of 12 N nitric acid was added dropwise thereto, and the mixture was stirred at 80°C for 8 hr for peptization. After the completion of the peptization, the mixture was filtered through a 0.45-µm filter and was subjected to solution exchange with a desalination column (PD-10, manufactured by Amersham Pharmacia Bioscience) to prepare an acidic titanium oxide sol having a solid content of 1 %. This titanium oxide sol was placed in a 100-ml vial bottle and was ultrasonicated at 200 kHz for 30 min. The average dispersed particle diameter after the ultrasonication was measured by a dynamic light scattering method. This measurement was carried out by diluting the ultrasonicated dispersion with 12 N nitric acid by a factor of 1000, charging 0.1 ml of the dispersion liquid in a quartz measurement cell, setting various parameters of the solvent to the same values as water, and measuring the dispersed particle diameter at 25°C with Zetasizer Nano ZS (manufactured by SYSMEX CORPORATION). As a result, it was found that the dispersed particle diameter was 20.2 nm.

This acidic titanium oxide sol (3 ml) thus obtained was dispersed in 20 ml of dimethylformamide (DMF). DMF (10 ml) containing 450 mg of polyethyleneimine having an average molecular weight of 10000 (manufactured by Wako Pure Chemical Industries, Ltd.) dissolved therein was added to the dispersion liquid, followed by stirring for mixing. The solution was transferred to a hydrothermal reaction vessel (HU-50 manufactured by SAN-Al science Co. Ltd.), and a reaction was allowed to proceed at 150°C for 5 hr. After the completion of the reaction, the reaction solution was cooled so that the temperature of the reaction vessel reached 50°C or below. Acetone in an amount of twice the amount of the reaction solution was added to the reaction solution. The mixture was allowed to stand at room temperature for 30 min and was then centrifuged under conditions of 2000 g and 15 min to recover precipitate. The surface of the recovered precipitate was washed with ethanol, and 1.5 ml of water was added thereto to give a dispersion liquid of polyethyleneimine-bound titanium oxide nanoparticles. This dispersion liquid was diluted with distilled water by a factor of 100, and the dispersed particle diameter and zeta potential were measured by a dynamic light scattering method. This measurement was carried out with Zetasizer Nano ZS by charging 0.75 ml of the dispersion liquid of the polyethyleneimine-bound titanium oxide nanoparticles in a zeta potential measuring cell, setting various parameters of the solvent to the same values as water, and conducting the measurement at 25°C. As a result, the dispersed particle diameter and the zeta potential were 57.5 nm and 47.5 mV, respectively.

Next, 5 ml of this dispersion liquid was subjected to gel filtration with a desalination column PD-10 equilibrated with a 50 mM borate buffer solution (pH 9.0) to conduct solution exchange with a 50 mM borate buffer solution (pH 9.0). To this collected solution was added 2.5 ml of an aqueous solution of a polyethylene glycol derivative (SUNBRIGHT ME-200CS (manufactured by NOF Co., Ltd.)) regulated to 20 mg/ml. The mixture was gently stirred at room temperature for 6 hr. This solution was concentrated to a volume of 1 ml with VIVAPORE 7500 (manufactured by VIVASCIENCE). This concentrate was further subjected to HPLC [AKTA Purifier (manufactured by Amersham Biosciences), column: HiPrep 16/60 Sephacryl S-300HR (manufactured by Amersham Biosciences), mobile phase: phosphate buffer solution (pH 7.4), flow rate: 0.3 ml/min]. As a result, an UV absorption peak was observed in a fraction passed through the column, and this fraction was recovered. The dispersed particle diameter and zeta potential of the collected fraction were measured by a dynamic light scattering method. This measurement was carried out with Zetasizer Nano ZS by charging 0.75 ml of the collected fraction into a zeta potential measuring cell, setting various parameters of the solvent to the same values as water, and conducting the measurement at 25°C. As a result of a cumulant analysis, it was found that the dispersed particle diameter and the zeta potential were 57.6 nm and 21.1 mV, respectively. The above results show that the introduction of polyethylene glycol into the polyethyleneimine-bound titanium oxide nanoparticles (TiO₂/PEG) reduced the surface charge.

### Example 4: Evaluation of photocatalytic activity of polyethylene glycol-bound titanium oxide nanoparticles

Each of the TiO₂/PEG dispersion liquids prepared in Examples 1 to 3 was diluted with PBS to a solid component content of 0.01 (w/v)%. Methylene blue trihydrate (manufactured by Wako Pure Chemical Industries, Ltd.) was added to the TiO₂/PEG-containing PBS solution prepared above to a concentration of 5 µM. The solution was irradiated with ultraviolet light with a wavelength of 340 nm at an exposure of 5 J/cm² with stirring, and the absorption at a wavelength of 660 nm was measured with an ultraviolet-visible spectrophotometer. The relative level (%) of the absorbance upon the decomposition of methylene blue in each sample was calculated as percentage methylene blue decomposition (%) by presuming the absorbance of a sample not irradiated with ultraviolet light to be 100%. The results are shown in Fig. 2. As shown in Fig. 2, for all the samples irradiated with ultraviolet light, a reduction in absorbance upon the decomposition of methylene blue was observed. This fact demonstrates that TiO₂/PEG prepared in Examples 1 to 3 had photocatalytic activity.

### Example 5: Evaluation of salt strength stability of polyethylene glycol-bound titanium oxide nanoparticles

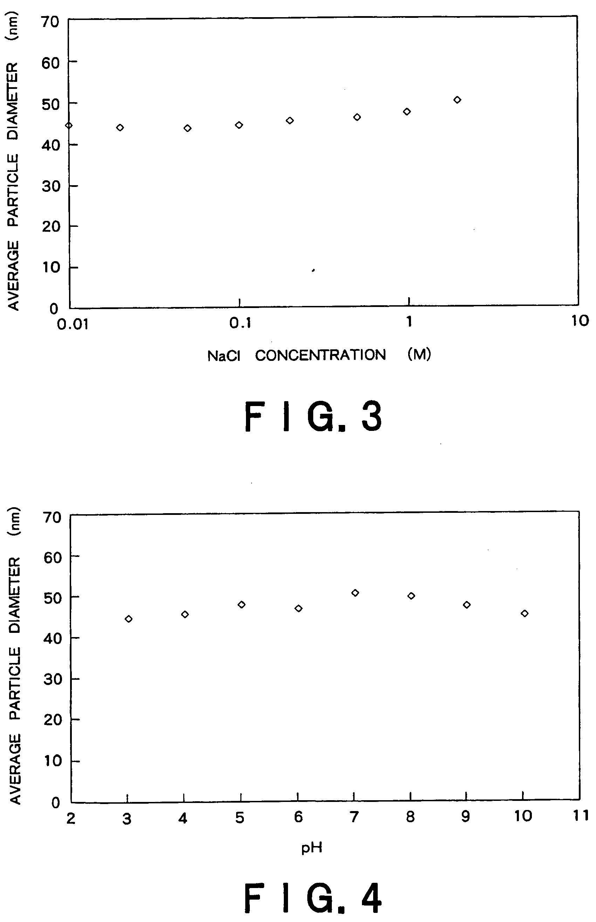

The TiO₂/PEG-containing dispersion liquid prepared in Example 1 was added to aqueous solutions containing sodium chloride in different concentrations of 0.01 to 2 M to a final concentration of 0.025%, and the mixture was allowed to stand at room temperature for one hr. Thereafter, the average dispersed particle diameter was measured with Zetasizer Nano ZS in the same manner as in Example 1. The results are shown in Fig. 3. As shown in Fig. 3, when the salt concentration of the system was between 0.01 M and 2 M, there was substantially no change in average dispersed particle diameter and the dispersibility was stable.

### Example 6: Evaluation of pH stability of polyethylene glycol-bound titanium oxide nanoparticles

The following buffer solutions (50 mM) having different pH values were prepared.
pH 3: Glycine hydrochloric acid buffer solution
pH 4 and 5: Acetate buffer solution
pH 6: 2-Morpholinoethanesulfonate buffer solution
pH 7 and 8: 2-[4-(2-Hydroxyethyl)-1-piperazinyl]ethanesulfonate buffer solution
pH 9: Borate buffer solution
pH10: Glycine sodium hydroxide buffer solution

The TiO₂/PEG-containing dispersion liquid prepared in Example 1 was added to these buffer solutions to a final concentration of 0.025 (w/v)%, and the mixture was allowed to stand at room temperature for one hr. Thereafter, the average dispersed particle diameter was measured with Zetasizer Nano ZS in the same manner as in Example 1. The results are shown in Fig. 4. As is apparent from Fig. 4, when the pH value was between 3 and 10, there was substantially no change in particle diameter and the dispersibility was stable.

### Example 7: Evaluation of dispersion stability of polyethylene glycol-bound titanium oxide nanoparticles in serum-added medium

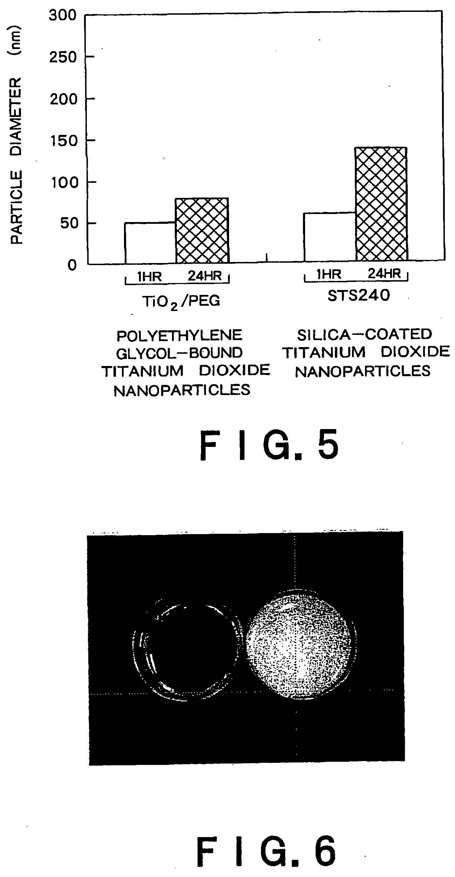

The TiO₂/PEG-containing dispersion liquid prepared in Example 1 and silica coated titanium oxide nanoparticles STS 240 (manufactured by Ishihara Sangyo Kaisha Ltd., dispersed particle diameter 52 nm) each were added to a 10% serum-containing RPMI 1640 medium (manufactured by GIBCO) to a final concentration of 0.025%, and the mixtures were allowed to stand at room temperature for one hr and 24 hr. Thereafter, the average dispersed particle diameter was measured with Zetasizer Nano ZS in the same manner as in Example 1. The results are shown in Fig. 5. After standing for 24 hr, there was substantially no change in average dispersed particle diameter of TiO₂/PEG, but on the other hand, the average dispersed particle diameter of the titanium oxide particles (A) underwent a significant change. Further, after standing for 72 hr, the silica-coated titanium oxide nanoparticles STS 240 formed precipitates while the average dispersed particle diameter of TiO₂/PEG was 80 nm. This fact demonstrates that the dispersion stability of TiO₂/PEG in the serum-added medium was good.

### Example 8: Evaluation of homogeneity (transparency) of polyethylene glycol-bound titanium oxide nanoparticles

The TiO₂/PEG-containing dispersion liquid prepared in Example 1 was diluted with a 0.1 M sodium chloride-containing 10 mM phosphate buffer solution to a final concentration of 0.1%, and the mixture was allowed to stand at room temperature for one hr. Separately, titanium oxide nanoparticles P25 (manufactured by Nippon Aerosil Co., Ltd.) were diluted with a 10 mM phosphate buffer solution containing 0.1 M sodium chloride to a final concentration of 0.1% in the same manner as described above, and the mixture was allowed to stand at room temperature for one hr. Thereafter, the diluted solutions were transferred respectively to 5-ml Petri dishes which were then photographed from above the dishes for observation. The results are shown in Fig. 6. As compared with the aqueous P25 solution indicated on the right side in the drawing, the TiO₂/PEG-containing dispersion liquid shown on the left side in the drawing apparently had a higher level of transparency and was in a homogeneously dispersed state. Further, the absorbance at a wavelength of 660 nm was measured with a spectrophotometer (UV-1600, manufactured by Shimadzu Seisakusho Ltd.). As a result, the absorbance of the aqueous P25 solution was much higher than 1 and was immeasurable. On the other hand, the TiO₂/PEG-containing dispersion liquid had an absorbance of 0.042, and the precipitate formation did not take place. Further, these solutions were allowed to stand at room temperature in a dark place for 2 weeks, and the absorbance was measured at a wavelength of 660 nm in the same manner as described above. As a result, the absorbance of the aqueous P25 solution was much higher than 1 and was immeasurable. On the other hand, the TiO₂/PEG-containing dispersion liquid had an absorbance of 0.052. The above fact demonstrates that, in the aqueous solution, the dispersion liquid of TiO₂/PEG had a higher level of transparency and more homogeneous dispersibility and, at the same time, was stable.

### Example 9: Evaluation of cytotoxity

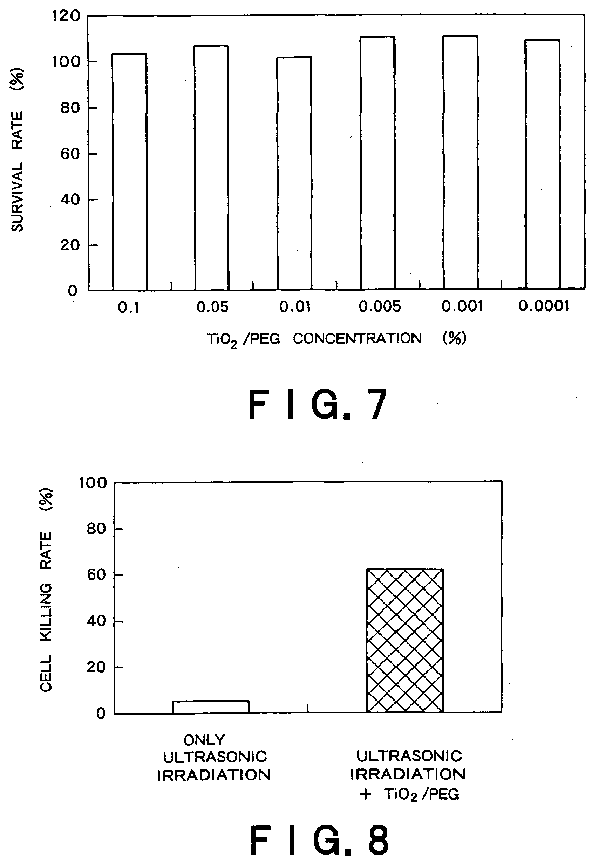

The TiO₂/PEG-containing dispersion liquid prepared in Example 1 was diluted with a 10% serum-containing RPMI 1640 medium (manufactured by GIBCO) to a solid content of 1.0%. Culture cancer cells (Jurkat) were cultured in a 10% serum-containing RPMI 1640 medium (manufactured by GIBCO) at 37°C under a 5% carbon dioxide atmosphere to prepare a cell culture having a concentration of 5.0 × 10⁴ cells/ml. This cell culture was again cultured for 20 hr under the same conditions. This cell culture was diluted with the TiO₂/PEG-containing dispersion liquid to final concentrations of 0.1%, 0.01%, 0.001%, and 0.0001% on a 96-hole plate to prepare a 200-µl cell culture solution for a test. This cell culture for a test was cultured at 37°C under a 5% carbon dioxide atmosphere for 20 hr. Each culture (100 µl) was used for a viable cell-derived luminous reaction by Celltiter-Glo Luminescent Cell Viability Assay (manufactured by Promega). The cytotoxity was evaluated by measuring the luminescence level with an image analyzer LAS-3000 UVmini (manufactured by Fuji Photo Film Co., Ltd.). Further, the relative level (%) of the luminescence level in each sample was calculated as the survival rate (%) by presuming the luminescence level in culture cells of an additive-free control to be 100%. The results are shown in Fig. 7. As shown in Fig. 7, for all the dispersion liquid concentrations, the same luminescence level, that is, the same survival rate, was confirmed, indicating that the dispersion liquid containing TiO₂/PEG in this concentration range did not have any cytotoxity.

### Example 10: Labelling of polyethylene glycol-bound titanium oxide nanoparticles with fluorescent dye

250 µl of 0.8 M 1-ethyl-3-[3-dimethylaminopropyl] carbodiimide hydrochloride and 250 µl of N-hydroxysuccinimide were added to 2 ml of this dispersion liquid of TiO₂/PEG prepared in Example 1. The mixture was allowed to react with stirring at room temperature for one hr. The reaction solution was subjected to solution exchange by gel filtration with a desalination column (PD-10, manufactured by Amersham Pharmacia Bioscience) equilibrated with a 10 mM acetate buffer solution (pH 5.0). Thereafter, a 10 mM acetate buffer solution (pH 5.0) was added thereto to give a total volume of 9.5 ml. To the mixture was added 5 µl of 100 mM 5-aminofluorescein (manufactured by NCI) dissolved in dimethylsulfoxide. The mixture was allowed to react at room temperature for one hr with stirring under light shielding conditions. Next, 500 µl of a 0.1 M aqueous solution of ethanolamine (manufactured by Wako Pure Chemical Industries, Ltd.) was added thereto, and the mixture was then allowed to react at room temperature with stirring under light shielding conditions for 30 min. This solution was subjected to solution exchange by gel filtration with a desalination column PD-10 equilibrated with 100 mM phosphate buffer brine (pH 7.5) to separate unreacted 5-aminofluorescein and thus to prepare a dispersion liquid containing a fluorescent dye-labeled TiO₂/PEG. The fluororescence intensity of this dispersion liquid and 5-aminofluorescein was measured with a fluorescence intensity measuring meter Fluoroskan Ascent CF (manufactured by Thermo Lasystems). As a result, in the dispersion liquid containing fluorescent dye-labeled TiO₂/PEG, a fluorescence intensity corresponding to 1.85 µm in terms of 5-aminofluorescein was confirmed. Further, the dry weight was measured. As a result, the titanium oxide nanoparticle solid content of this dispersion liquid was 0.32 (w/v)%. Based on this, the content of the carboxyl group per unit weight of TiO₂/PEG was determined. As a result, the carboxyl group content/titanium oxide nanoparticle content ratio in the dispersion liquid was 5.8 × 10⁻⁷ (mol/g).

### Example 11: Test on cell killing by polyethylene glycol-bound titanium oxide nanoparticles and ultrasonic irradiation

TiO₂/PEG prepared in Example 1 was dispersed in a PBS buffer solution (pH 7.4) to a final concentration of 0.05%. This solution was added in an amount of 1/10 to a 10% serum-added RPMI 1640 medium (manufactured by Invitrogen) containing 1 × 10⁴ cells/ml Jurkat cells to prepare a test solution. The test solution was exposed to ultrasonic waves from an ultrasonic irradiation apparatus (ULTRASONIC APPARATUS ES-2: 1 MHz, manufactured by OG GIKEN CO., LTD.) under conditions of 0.5 W/cm² and 50% duty cycle for one min to determine the cell kill rate (%). For comparison, the same test was carried out except that TiO₂/PEG was not used. The results are shown in Fig. 8. As shown in Fig. 8, the kill rate of cells, which was very low when TiO₂/PEG was not used, was brought to very high when TiO₂/PEG was used. Accordingly, it could be confirmed that cells could be killed with high efficiency by ultrasonic irradiation in the presence of TiO₂/PEG.

### Example 12: Preparation of titanium dioxide sol

Titanium tetraisopropoxide (3.6 g) was mixed with 3.6 g of isopropanol, and the mixture was added dropwise to 60 ml of ultrapure water under ice cooling for hydrolysis. After the dropwise addition, the mixture was stirred at room temperature for 30 min. After the stirring, 1 ml of 12 N nitric acid was added dropwise thereto, and the mixture was stirred at 80°C for 8 hr for peptization. After the completion of the peptization, the mixture was filtered through a 0.45-µm filter and was subjected to solution exchange with a free-fall column PD-10 (manufactured by GE Healthcare Bioscience) for buffer exchange to prepare an acidic titanium dioxide sol having a solid content of 1%. The titanium dioxide sol was placed in a 100-ml vial bottle and was ultrasonicated at 200 Hz for 30 min in an ultrasonic generator MIDSONIC 200 (manufactured by KAIJO Corporation). The average dispersed particle diameter after the ultrasonication was measured by diluting the ultrasonicated titanium oxide sol with 12 N nitric acid by a factor of 1000, charging 0.1 ml of the dispersion liquid in a quartz measurement cell, setting various parameters of the solvent to the same values as water, and measuring the dispersed particle diameter at 25°C with Zetasizer Nano ZS (manufactured by SYSMEX CORPORATION). As a result, it was found that the dispersed particle diameter was 20.2 nm. The titanium oxide sol solution was concentrated at a temperature of 50°C using an evaporating dish to finally prepare an acidic titanium oxide sol having a solid content of 20%. The average dispersed particle diameter after the ultrasonication was measured by a dynamic light scattering method.

### Example 13: Introduction of polyethylene glycol into titanium dioxide particles

Water (10 ml) was added to 1 g of a polymerization initiator VPE-0201 (molecular weight of polymeric initiator Mn = about 15000 to 30000, manufactured by Wako Pure Chemical Industries, Ltd.) comprising a plurality of polyethylene oxides bound to each other through an azo group to prepare a polyethylene oxide polymerization initiator solution (100 mg/ml). A dimethylformamide (DMF: manufactured by Wako Pure Chemical Industries, Ltd.) solution (10 ml) was added to 0.15412 g of protocatechuic acid (molecular weight Mn = 154.12: manufactured by Wako Pure Chemical Industries, Ltd.) as a ligand molecule to prepare a 100 mM protocatechuic acid solution. The anatase-type titanium dioxide sol (0.25 ml) prepared in Example 12 was added to 5.75 ml of DMF. The protocatechuic acid solution (1.5 ml) and 3 ml of a polyethylene oxide polymerization initiator solution were added thereto, followed by stirring for mixing. Thereafter, the solution was transferred to a hydrothermal reactor HU-50 (manufactured by SAN-Al Science Co. Ltd.), and a hydrothermal synthesis was allowed to proceed at 80°C for 16 hr. After the completion of the reaction, the reaction solution was cooled to a reaction vessel temperature of 50°C or below. A phosphate buffer solution (PBS: pH 7.4) (9 ml) was added to 1 ml of the solution after the reaction to prepare a PBS diluted solution. The diluted solution (2.5 ml) was collected with 3.5 ml of a PBS solution through a desalination column PD-10 (manufactured by GE Healthcare Bioscience), and the organic solvent was removed to prepare a dispersion liquid of titanium oxide composite particles.

The dispersed particle diameter of the titanium oxide composite particles was measured with Zetasizer Nano ZS (manufactured by SYSMEX CORPORATION). This measurement was carried out by charging 0.75 ml of a dispersion liquid of polyethylene glycol-bound titanium dioxide nanoparticles in a zeta potential measuring cell, setting various parameters of the solvent to the same values as water, and measuring the particle diameter by a dynamic light scattering method at 25°C. As a result, the average particle diameter of the polyethylene glycol-bound titanium dioxide nanoparticles was 27.3 nm. Further, the zeta potential was measured with Zetasizer Nano ZS under the same conditions. As a result, the zeta potential of the polyethylene glycol-bound titanium dioxide nanoparticles was -9.27 mV.

### Example 14: Introduction of polyethylene glycol into protocatechuic acid-bound titanium dioxide nanoparticles

Dimethylformamide (DMF: manufactured by Wako Pure Chemical Industries, Ltd.) solution (10 ml) was added to 0.15412 g of protocatechuic acid (molecular weight Mn = 154.12: manufactured by Wako Pure Chemical Industries, Ltd.) as a ligand molecule to prepare a 100 mM protocatechuic acid solution. The anatase-type titanium dioxide sol (0.25 ml) prepared in Example 12 was added to 9.25 ml of DMF. The protocatechuic acid solution (0.5 ml) was added thereto, followed by stirring for mixing. Thereafter, the solution was transferred to a hydrothermal reactor HU-50 (manufactured by SAN-Al Science Co. Ltd.), and a hydrothermal synthesis was allowed to proceed at 150°C for 16 hr. After the completion of the reaction, the reaction solution was cooled to a reaction vessel temperature of 50°C or below, and 20 ml of isopropanol in an amount of twice the amount of the reaction solution was added. The mixture was allowed to stand at room temperature for 30 min and was then centrifuged at 2000 g x 15 min to collect precipitates. The surface of the collected precipitates was washed with ethanol. A 50 mM borate buffer solution (pH 9) (10 ml) was added thereto to prepare a dispersion liquid of 0.5 (wt/vol)% protocatechuic acid-bound titanium dioxide nanoparticles. This dispersion liquid was diluted with distilled water by a factor of 10. The dispersed particle diameter and zeta potential of the diluted solution were measured by a dynamic light scattering method with Zetasizer Nano ZS. Specifically, they were measured by charging 0.75 ml of the dispersion liquid of the protocatechuic acid-bound titanium dioxide nanoparticles in a zeta potential measuring cell, setting various parameters of the solvent to the same values as water, and conducting the measurement at 25°C. As a result, the dispersed particle diameter and the zeta potential were 30.3 nm and -22.6 mV, respectively.

Next, water (10 ml) was added to 1 g of a polymerization initiator VPE-0201 (molecular weight of polymeric initiator Mn = about 15000 to 30000, manufactured by Wako Pure Chemical Industries, Ltd.) comprising a plurality of polyethylene oxides bound to each other through an azo group to prepare a polyethylene oxide polymerization initiator solution (100 mg/ml). The polyethylene oxide polymerization initiator solution (3 ml) and 3 ml of a 50 mM borate buffer solution were added to 4 ml of the 0.5 (wt/vol)% protocatechuic acid-bound titanium dioxide solution, followed by stirring for mixing. Thereafter, the solution was transferred to a hydrothermal reaction vessel HU-50 (manufactured by SAN-Al Science Co. Ltd.), and a synthesis reaction was allowed to proceed at 60°C for 16 hr. After the completion of the reaction, the reaction solution was cooled to a reaction vessel temperature of 50°C or below. The solution (2.5 ml) after the reaction was subjected to solution exchange through a free-fall column PD-10 (manufactured by GE Healthcare Bioscience) for buffer exchange and collected with 3.5 ml of water to prepare solution exchanged dispersion liquid of titanium oxide composite particles. The dispersed particle diameter of the titanium oxide composite particles was measured with Zetasizer Nano ZS (manufactured by SYSMEX CORPORATION). This measurement was carried out by charging 0.75 ml of the dispersion liquid of titanium oxide composite particles in a zeta potential measuring cell, setting various parameters of the solvent to the same values as water, and measuring the particle diameter by a dynamic light scattering method at 25°C. As a result, the average particle diameter of the titanium oxide composite particles was 42.2 nm. Further, the zeta potential was measured with Zetasizer Nano ZS under the same conditions. As a result, the zeta potential of the titanium oxide composite particles was -10.8 mV.

### Example 15: Introduction of polyethylene glycol into gallic acid-bound titanium dioxide nanoparticles

Dimethylformamide (DMF: manufactured by Wako Pure Chemical Industries, Ltd.) solution (10 ml) was added to 0.1701 g of gallic acid (molecular weight Mn = 170.1: manufactured by Wako Pure Chemical Industries, Ltd.) as a ligand molecule to prepare a 100 mM gallic acid solution. The anatase-type titanium dioxide sol (0.25 ml) prepared in Example 12 was added to 9.25 ml of DMF. The gallic acid solution (0.5 ml) was added thereto, followed by stirring for mixing. Thereafter, the solution was transferred to a hydrothermal reactor HU-50 (manufactured by SAN-Al Science Co. Ltd.), and a hydrothermal synthesis was allowed to proceed at 150°C for 16 hr. After the completion of the reaction, the reaction solution was cooled to a reaction vessel temperature of 50°C or below, and 20 ml of isopropanol in an amount of twice the amount of the reaction solution was added. The mixture was allowed to stand at room temperature for 30 min and was then centrifuged at 2000 g x 15 min to collect precipitates. The surface of the collected precipitates was washed with ethanol. Water (10 ml) was added thereto to prepare a dispersion liquid of 0.5 (wt/vol)% gallic acid-bound titanium dioxide nanoparticles. This dispersion liquid was diluted with distilled water by a factor of 10. The dispersed particle diameter and zeta potential of the diluted solution were measured by a dynamic light scattering method with Zetasizer Nano ZS. Specifically, they were measured by charging 0.75 ml of the dispersion liquid of the gallic acid-bound titanium dioxide nanoparticles in a zeta potential measuring cell, setting various parameters of the solvent to the same values as water, and conducting the measurement at 25°C. As a result, the dispersed particle diameter and the zeta potential were 32.6 nm and -36.0 mV, respectively.

Next, water (10 ml) was added to 1 g of a polymerization initiator VPE-0201 (molecular weight of polymeric initiator Mn = about 15000 to 30000, manufactured by Wako Pure Chemical Industries, Ltd.) comprising a plurality of polyethylene oxides bound to each other through an azo group to prepare a polyethylene oxide polymerization initiator solution (100 mg/ml). An aqueous solution (pH 5.5) (3 ml) prepared from 3 ml of the polyethylene oxide polymerization initiator solution and hydrochloric acid was added to 4 ml of the 0.5 (wt/vol)% gallic acid-bound titanium dioxide solution, followed by stirring for mixing. Thereafter, the solution was transferred to a hydrothermal reaction vessel HU-50 (manufactured by SAN-Al Science Co. Ltd.), and a synthesis reaction was allowed to proceed at 60°C for 16 hr. After the completion of the reaction, the reaction solution was cooled to a reaction vessel temperature of 50°C or below. The solution (2.5 ml) after the reaction was subjected to solution exchange through a free-fall column PD-10 (manufactured by GE Healthcare Bioscience) for buffer exchange and collected with 3.5 ml of water to prepare solution exchanged dispersion liquid of titanium oxide composite particles. The dispersed particle diameter of the titanium oxide composite particles was measured with Zetasizer Nano ZS (manufactured by SYSMEX CORPORATION). This measurement was carried out by charging 0.75 ml of the dispersion liquid of titanium oxide composite particles in a zeta potential measuring cell, setting various parameters of the solvent to the same values as water, and measuring the particle diameter by a dynamic light scattering method at 25°C. As a result, the average particle diameter of the titanium oxide composite particles was 42.5 nm. Further, the zeta potential was measured with Zetasizer Nano ZS under the same conditions. As a result, the zeta potential of the titanium oxide composite particles was -20.0 mV.

### Example 16: Evaluation on polymer bonding amount in polyethylene glycol-bound titanium dioxide nanoparticles

The following particles were dispersed in distilled water to a solid content of 0.2 (w/v)% to prepare a sample.
· TiO₂/PEG (A): Titanium oxide composite particles prepared in Example 14
· TiO₂/PEG (B): Titanium oxide composite particles prepared in the same manner as in Example 3 except that a 33 mg/ml polyethylene oxide polymerization initiator solution was used.
TiO₂/PEG (C): Titanium oxide composite particles prepared in Example 15
TiO₂/PEG (D): Titanium oxide composite particles prepared in the same manner as in Example 4 except that a 33 mg/ml polyethylene oxide polymerization initiator solution was used

Acetone (manufactured by Wako Pure Chemical Industries, Ltd.) (20 ml) and 0.5 ml of a 5 M aqueous sodium chloride solution were added to 5 ml of the aqueous solution. The mixture was thoroughly stirred to form precipitates and was then centrifuged, and the supernatant was then removed. A procedure consisting of adding 5 ml of distilled water to the precipitates to prepare a mixture, adding acetone and a 5 M aqueous sodium chloride solution to the mixture in the same manner as described above and centrifuging the mixture was repeated three times. Next, 5 ml of distilled water was added to the collected precipitates, and this solution was desalinated by gel filtration with a desalination column NAP-10 (manufactured by GE Healthcare Bioscience) equilibrated with distilled water. The treated solution was transferred to a ceramic evaporating dish and was dried in an electrothermic drier at 100°C for 16 hr to prepare a dry powder. The dry powder was heated in air with a differential thermal/thermogravimetric simultaneous measuring apparatus (EXSTAR 6300: manufactured by SII) at 100°C for 30 min and was then heated at 600°C for 30 min to measure a weight change. The results are shown in Table 1. The weight change up to 600°C after the complete removal of water at 100°C was considered attributable to the combustion of polyethylene glycol, and, based on these data, the polymer bonding amount per unit titanium amount of the titanium oxide composite particles was obtained.

**[Table 1]**

| | Polymer bonding amount per unit titanium amount (g/g) |
|---|---|
| TiO₂/PEG (A) | 0.45 |
| TiO₂/PEG (B) | 0.32 |
| TiO₂/PEG (C) | 0.47 |
| TiO₂/PEG (D) | 0.34 |

### Example 17: Introduction of methyldopa-bound polyethylene glycol into titanium dioxide particles

Water (10 ml) was added to 1 g of a polymerization initiator VPE-0401 (molecular weight of polymeric initiator Mn = about 25000 to 40000, manufactured by Wako Pure Chemical Industries, Ltd.) comprising a plurality of polyethylene oxides bound to each other through an azo group to prepare a polyethylene oxide polymerization initiator solution (100 mg/ml). Water (10 ml) was added to 211 mg of methyldopa (3-(3,4-dihydroxyphenyl)-2-methyl-L-alanine; molecular weight Mn = 211.2; manufactured by Tokyo Chemical Industry Co., Ltd.) as a ligand molecule to prepare a 100 mM methyldopa solution. The methyldopa solution was mixed with a polyethylene oxide polymerization initiator solution to prepare 10 ml of an aqueous mixed solution so that the final concentration of methyldopa and the final concentration of polyethylene oxide were 10 mM and 50 mg/ml, respectively. Thereafter, the solution was transferred to a hydrothermal reaction vessel HU-50 (manufactured by SAN-Al Science Co. Ltd.) and was heated at 60°C for 16 hr. After the completion of the reaction, the reaction solution was cooled to a reaction vessel temperature of 50°C or below. The whole cooled solution was transferred to an egg-plant type flask and was lyophilized overnight to prepare 510 mg of methyldopa-bound polyethylene oxide powder. Dimethylformamide (DMF: manufactured by Wako Pure Chemical Industries, Ltd.) (6 ml) was added to and mixed with this powder. Further, this mixed solution was regulated in DMF to prepare a reaction solution so that the final concentration was 40 (v/v)% and the final concentration of the anatase-type titanium dioxide sol prepared in Example 12 was 0.2% in terms of solid content. This reaction solution was transferred to the hydrothermal reaction vessel HU-50 and was thermally reacted at 80°C for 16 hr. After the completion of the reaction, the reaction solution was cooled to a reaction vessel temperature of 50°C or below. DMF was removed from the reaction solution by an evaporator. Distilled water (10 ml) was added to the residue to prepare a dispersion liquid of titanium oxide composite particles.

Further, the dispersion liquid thus obtained was subjected to HPLC under the following conditions. As a result, an UV absorption peak was observed in a fraction passed through the column, and this fraction was recovered.
- Apparatus: AKTA Purifier (manufactured by GE Healthcare Bioscience)
- Column: HiPrep 16/60 Sephacryl S-300HR (manufactured by GE Healthcare Bioscience)
- Mobile phase: Phosphate buffer solution (pH 7.4)
- Flow rate: 0.3 ml/min

The dispersion liquid was diluted with distilled water to prepare a 0.01% aqueous solution, and the dispersed particle diameter and zeta potential were measured by a dynamic light scattering method. This measurement was carried out with Zetasizer Nano ZS by charging 0.75 ml of a dispersion liquid of polyethylene glycol-bound titanium dioxide nanoparticles into a zeta potential measuring cell, setting various parameters of the solvent to the same values as water, and conducting the measurement at 25°C. As a result of a cumulant analysis, it was found that the dispersed particle diameter and the zeta potential were 37.4 nm and -5.3 mV, respectively.

### Example 18: Introduction of polyethylene glycol into guinic acid-bound titanium dioxide nanoparticles

Dimethylformamide (DMF: manufactured by Wako Pure Chemical Industries, Ltd.) solution (10 ml) was added to 0.1922 g of quinic acid (molecular weight Mn = 192.2: manufactured by MP Biomedicals, Inc.) as a ligand molecule to prepare a 100 mM quinic acid solution. The anatase-type titanium dioxide sol (0.25 ml) prepared in Example 12 was added to 9.25 ml of DMF. The quinic acid solution (0.5 ml) was added thereto, followed by stirring for mixing. Thereafter, the solution was transferred to a hydrothermal reactor HU-50 (manufactured by SAN-Al Science Co. Ltd.), and a hydrothermal synthesis was allowed to proceed at 150°C for 16 hr. After the completion of the reaction, the reaction solution was cooled to a reaction vessel temperature of 50°C or below, and 20 ml of isopropanol in an amount of twice the amount of the reaction solution was added. The mixture was allowed to stand at room temperature for 30 min and was then centrifuged at 2000 g x 15 min to collect precipitates. The surface of the collected precipitates was washed with ethanol. Water (10 ml) was added thereto to prepare a dispersion liquid of 0.5 (wt/vol)% quinic acid-bound titanium dioxide nanoparticles. This dispersion liquid was diluted with distilled water by a factor of 10. The dispersed particle diameter and zeta potential of the diluted solution were measured by a dynamic light scattering method with Zetasizer Nano ZS. Specifically, they were measured by charging 0.75 ml of the dispersion liquid of the quinic acid-bound titanium dioxide nanoparticles in a zeta potential measuring cell, setting various parameters of the solvent to the same values as water, and conducting the measurement at 25°C. As a result, the dispersed particle diameter was 29.3 nm.

Next, water (10 ml) was added to 1 g of a polymerization initiator VPE-0401 (molecular weight of polymeric initiator Mn = about 25000 to 40000, manufactured by Wako Pure Chemical Industries, Ltd.) comprising a plurality of polyethylene oxides bound to each other through an azo group to prepare a polyethylene oxide polymerization initiator solution (100 mg/ml). The polyethylene oxide polymerization initiator solution (3 ml) and 3 ml of a 50 mM borate buffer solution (pH 9) were added to 4 ml of the 0.5 (wt/vol)% quinic acid-bound titanium dioxide solution, followed by stirring for mixing. Thereafter, the solution was transferred to a hydrothermal reaction vessel HU-50 (manufactured by SAN-Al Science Co. Ltd.), and a synthesis reaction was allowed to proceed at 80°C for 16 hr. After the completion of the reaction, the reaction solution was cooled to a reaction vessel temperature of 50°C or below. The solution (2.5 ml) after the reaction was subjected to solution exchange through a free-fall column PD-10 (manufactured by GE Healthcare Bioscience) for buffer exchange and collected with 3.5 ml of water to prepare solution exchanged dispersion liquid of titanium oxide composite particles. The dispersed particle diameter of the polyethylene glycol-bound titanium dioxide nanoparticles was measured with Zetasizer Nano ZS (manufactured by SYSMEX CORPORATION). This measurement was carried out by charging 0.75 ml of the dispersion liquid of titanium oxide composite particles in a zeta potential measuring cell, setting various parameters of the solvent to the same values as water, and measuring the particle diameter by a dynamic light scattering method at 25°C. As a result, the average particle diameter of the titanium oxide composite particles was 178 nm. Further, the zeta potential was measured with Zetasizer Nano ZS under the same conditions. As a result, the zeta potential of the titanium oxide composite particles was -20.0 mV.

### Example 19: Introduction of polyethylene glycol into aminomethylphosphonic acid-bound titanium dioxide nanoparticles

A dimethylformamide (DMF: manufactured by Wako Pure Chemical Industries, Ltd.) solution (10 ml) was added to 0.111 g of aminomethylphosphonic acid (molecular weight Mn = 111.04: manufactured by Sigma) as a ligand molecule to prepare an aminomethylphosphonic acid solution (100 mM). The anatase-type titanium dioxide sol (0.25 ml) prepared in Example 12 was added to 9.25 ml of DMF. The aminomethylphosphonic acid solution (0.5 ml) was added thereto followed by stirring for mixing. Thereafter, the solution was transferred to a hydrothermal reactor HU-50 (manufactured by SAN-Al Science Co. Ltd.), and a hydrothermal synthesis was allowed to proceed at 150°C for 16 hr. After the completion of the reaction, the reaction solution was cooled to a reaction vessel temperature of 50°C or below, and 20 ml of isopropanol in an amount of twice the amount of the reaction solution was added. The mixture was allowed to stand at room temperature for 30 min and was then centrifuged at 2000 g x 15 min to collect precipitates. The surface of the collected precipitates was washed with ethanol. Water (10 ml) was added thereto to prepare a dispersion liquid of 0.5 (wt/vol)% aminomethylphosphonic acid-bound tintanium dioxide nanoparticles. This dispersion liquid was diluted with distilled water by a factor of 10. The dispersed particle diameter and zeta potential of the diluted solution were measured by a dynamic light scattering method with Zetasizer Nano ZS. Specifically, they were measured by charging 0.75 ml of the dispersion liquid of the gallic acid-bound titanium dioxide nanoparticles in a zeta potential measuring cell, setting various parameters of the solvent to the same values as water, and conducting the measurement at 25°C. As a result, the dispersed particle diameter and the zeta potential were 30.5 nm and -30.0 mV, respectively.

Next, water (10 ml) was added to 1 g of a polymerization initiator VPE-0201 (molecular weight of polymeric initiator Mn = about 15000 to 30000, manufactured by Wako Pure Chemical Industries, Ltd.) comprising a plurality of polyethylene oxides bound to each other through an azo group to prepare a polyethylene oxide polymerization initiator solution (100 mg/ml). An aqueous solution (pH 5.5) (3 ml) prepared from 3 ml of the polyethylene oxide polymerization initiator solution and hydrochloric acid was added to 4 ml of the 0.5 (wt/vol)% aminomethylphosphonic acid-bound titanium dioxide solution, followed by stirring for mixing. Thereafter, the solution was transferred to a hydrothermal reaction vessel HU-50 (manufactured by SAN-Al Science Co. Ltd.), and a synthesis reaction was allowed to proceed at 60°C for 16 hr. After the completion of the reaction, the reaction solution was cooled to a reaction vessel temperature of 50°C or below. The solution (2.5 ml) after the reaction was subjected to solution exchange through a free-fall column PD-10 (manufactured by GE Healthcare Bioscience) for buffer exchange and collected with 3.5 ml of water to prepare solution exchanged dispersion liquid of titanium oxide composite particles. The dispersed particle diameter of the titanium oxide composite particles was measured with Zetasizer Nano ZS (manufactured by SYSMEX CORPORATION). This measurement was carried out by charging 0.75 ml of the dispersion liquid of titanium oxide composite particles in a zeta potential measuring cell, setting various parameters of the solvent to the same values as water, and measuring the particle diameter by a dynamic light scattering method at 25°C. As a result, the average particle diameter of the titanium oxide composite particles was 50.0 nm. Further, the zeta potential was measured with Zetasizer Nano ZS under the same conditions. As a result, the zeta potential of the titanium oxide composite particles was -20.0 mV.

### Example 20: Introduction of polyethylene glycol into 4-aminosalicylic acid-bound titanium dioxide nanoparticles

A dimethylformamide (DMF: manufactured by Wako Pure Chemical Industries, Ltd.) solution (10 ml) was added to 0.15314 g of 4-aminosalicylic acid (molecular weight Mn = 153.14: manufactured by MP Biomedicals, Inc.) as a ligand molecule to prepare a 100 mM 4-aminosalicyclic acid solution. The anatase-type titanium dioxide sol (0.25 ml) prepared in Example 12 was added to 9.25 ml of DMF. The 4-aminosalicyclic acid solution (0.5 ml) was added thereto, followed by stirring for mixing. Thereafter, the solution was transferred to a hydrothermal reactor HU-50 (manufactured by SAN-Al Science Co. Ltd.), and a hydrothermal synthesis was allowed to proceed at 150°C for 16 hr. After the completion of the reaction, the reaction solution was cooled to a reaction vessel temperature of 50°C or below, and 20 ml of isopropanol in an amount of twice the amount of the reaction solution was added. The mixture was allowed to stand at room temperature for 30 min and was then centrifuged at 2000 g x 15 min to collect precipitates. The surface of the collected precipitates was washed with ethanol. Water (10 ml) was added thereto to prepare a dispersion liquid of 0.5 (wt/vol)% 4-aminosalicylic acid-bound titanium dioxide nanoparticles. This dispersion liquid was diluted with distilled water by a factor of 10. The dispersed particle diameter and zeta potential of the diluted solution were measured by a dynamic light scattering method with Zetasizer Nano ZS. Specifically, they were measured by charging 0.75 ml of the dispersion liquid of the 4-aminosalicylic acid-bound titanium dioxide nanoparticles in a zeta potential measuring cell, setting various parameters of the solvent to the same values as water, and conducting the measurement at 25°C. As a result, the dispersed particle diameter was 32.7 nm.

Next, 5 ml of this dispersion liquid was subjected to solution exchange with a 50 mM borate buffer solution (pH 9.0) by gel filtration with a free-fall column PD-10 (manufactured by GE Healthcare Bioscience), for buffer exchange, equilibrated with a 50 mM borate buffer solution (pH 9.0). An aqueous solutions (2.5 ml) of a polyethylene glycol derivative (SUNBRIGHT ME-200CS (manufactured by NOF Co., Ltd.)) having a concentration of 20 mg/ml was added to the collected solution, and the mixture was gently stirred at room temperature for 6 hr. This solution was concentrated to a volume of 1 ml with VIVAPORE 7500 (manufactured by VIVASCIENCE).

Further, the concentrate thus obtained was subjected to HPLC under the following conditions. As a result, an UV absorption peak was observed in a fraction passed through the column, and this fraction was recovered.
· Apparatus: AKTA Purifier (manufactured by GE Healthcare Bioscience)
· Column: HiPrep 16/60 Sephacryl S-300HR (manufactured by GE Healthcare Bioscience)
· Mobile phase: Phosphate buffer solution (pH 7.4)
· Flow rate: 0.3 ml/min

The dispersed particle diameter and zeta potential of the collected fraction were measured with Zetasizer Nano ZS (manufactured by SYSMEX CORPORATION). This measurement was carried out by charging 0.75 ml of the dispersion liquid of titanium oxide composite particles in a zeta potential measuring cell, setting various parameters of the solvent to the same values as water, and measuring the particle diameter by a dynamic light scattering method at 25°C. As a result, the average particle diameter of the titanium oxide composite particles was 45.9 nm. Further, the zeta potential was measured with Zetasizer Nano ZS under the same conditions. As a result, the zeta potential of the titanium oxide composite particles was -2.0 mV.

### Example 21: Evaluation on salt strength stability of polyethylene glycol-bound titanium dioxide nanoparticles

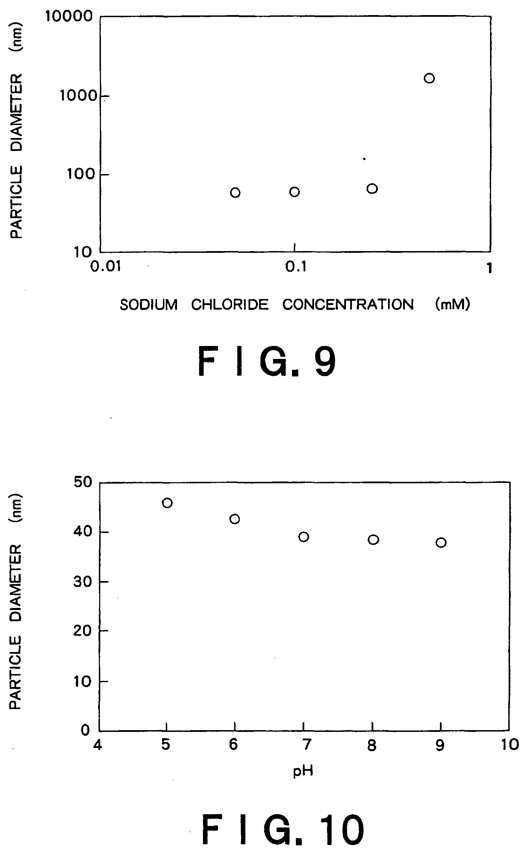

The titanium oxide composite particle-containing dispersion liquid prepared in Example 13 was added to aqueous solutions containing sodium chloride in different concentrations of 0.01 to 0.5 M to a final concentration of 0.025%, and the mixture was allowed to stand at room temperature for one hr. Thereafter, the average dispersed particle diameter was measured with Zetasizer Nano ZS in the same manner as in Example 1. The results are shown in Fig. 9. As shown in Fig. 9, when the salt concentration of the system was between 0.01 M and 0.25 M, there was substantially no change in average dispersed particle diameter and the dispersibility was stable.

### Example 22: Evaluation of pH stability of titanium oxide composite particles

The following 50 mM buffer solutions having different pH values were prepared. The titanium oxide composite particle-containing dispersion liquid prepared in Example 15 was added to the buffer solutions to a final concentration of 0.025 (w/v)%, and the mixture was allowed to stand at room temperature for one hr.
· pH 5: Acetate buffer solution
· pH 6: 2-Morpholinoethanesulfonate buffer solution
· pH 7 and pH 8: 2-[4-(2-Hydroxyethyl)-1-piperazinyl]ethanesulfonic acid buffer solution
· pH 9: Borate buffer solution

Thereafter, the average dispersed particle diameter was measured with Zetasizer Nano ZS in the same manner as in Example 12. The results are shown in Fig. 10. As shown in Fig. 10, it was found that, when the pH value was between 5 and 9, there was no substantial change in particle diameter and stable dispersibility could be realized.

### Example 23: Evaluation of dispersion stability of titanium oxide composite particles in protein solution

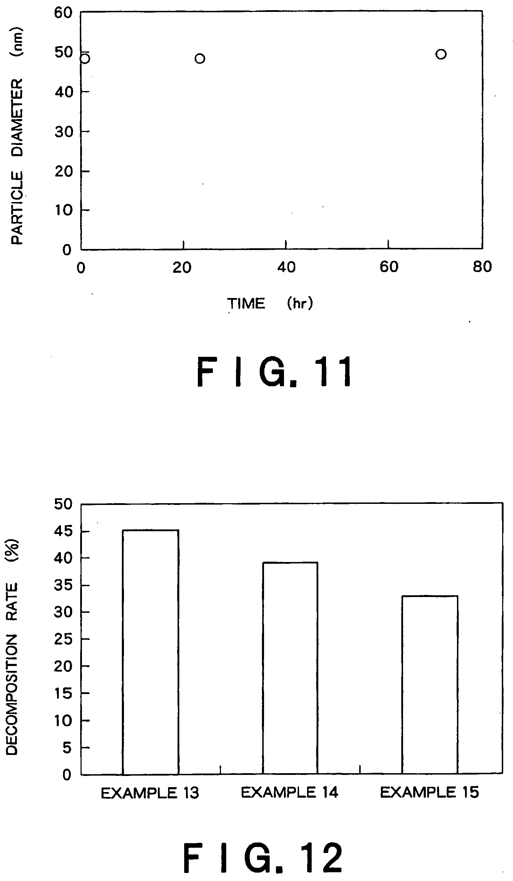

The titanium oxide composite particle-containing dispersion liquid prepared in Example 15 was added to a 10% serum-containing RPMI 1640 medium (manufactured by GIBCO) to a final concentration of 0.025%, and the mixture was allowed to stand at room temperature for one hr, 24 hr, and 72 hr. Thereafter, the average dispersed particle diameter was measured with Zetasizer Nano ZS in the same manner as in Example 1. The results are shown in Fig. 11. As shown in Fig. 11, after standing for 72 hr, there was no substantial change in particle diameter of the titanium oxide composite particles, demonstrating that stable dispersibility was realized.

### Example 24: Evaluation on photocatalytic activity of titanium oxide composite particles

Each of the titanium oxide composite particles prepared in Examples 13 to 15 was diluted with PBS to a solid content of 0.01 (w/v)%. Methylene blue trihydrate (manufactured by Wako Pure Chemical Industries, Ltd.) was added to the titanium oxide composite particle-containing PBS solution prepared above to a concentration of 5 µM. The solution was irradiated with ultraviolet light with a wavelength of 340 nm at an exposure of 5 J/cm² with stirring, and the absorption at a wavelength of 660 nm was measured with an ultraviolet-visible spectrophotometer. The results are shown in Fig. 12. As shown in Fig. 12, when the absorbance of a sample not irradiated with ultraviolet light was presumed to be 100%, for all the irradiated samples, there was a reduction in absorbance attributable to the decomposition of the methylene blue, demonstrating that the titanium oxide composite particles prepared in Examples 13 to 15 had photocatalytic activity.

### Example 25: Introduction of 4-aminosalicylic acid-bound polyethylene glycol into titanium dioxide particles

Water (5 ml) was added to 1 g of a copolymer of polyoxyethylene-monoallyl-monomethyl ether with maleic anhydride (average molecular weight; 33659, manufactured by NOF Co., Ltd.), and the mixture was hydrolyzed. The solution thus obtained and 1-ethyl-3-(3-dimethylaminopropyl)carbodiimide hydrochloride (manufactured by DOJINDO LABORATORIES) were regulated with ultrapure water while mixing to concentrations of 50 mg/ml and 50 mM, respectively. 4-Aminosalicylic acid (molecular weight Mn = 153.14: MP Biomedicals, Inc.) was mixed with the solution to a concentration of 100 mM to prepare a 4-ml solution. This solution was allowed to react at room temperature with stirring by shaking for 72 hr. After the completion of the reaction, the solution was transferred to a Spectra/Pore CE dialysis tube (cut-off molecular weight = 3500, manufactured by Spectrum Laboratories, Inc.) as a dialytic membrane and was dialyzed against 4 liters of ultrapure water at room temperature for 24 hr. After the dialysis, the whole solution was transferred to an egg-plant type flask and was lyophilized overnight, and 4 ml of dimethylformamide (DMF: manufactured by Wako Pure Chemical Industries, Ltd.) was added to and mixed with the resultant powder to prepare a 4-aminosalicylic acid-bound polyethylene glycol solution.

The 4-aminosalicylic acid-bound polyethylene glycol solution and the anatase-type titanium dioxide sol prepared in Example 12 were then regulated with DMF to a final concentration of 20 (vol/vol)% and a final concentration (solid content) of 0.25%, respectively, to prepare a 2.5-ml reaction solution. This reaction solution was transferred to a hydrothermal reaction vessel (HU-50, manufactured by SAN-AI Science Co. Ltd.), and a thermal reaction was allowed to proceed at 80°C for 6 hr. After the completion of the reaction, the reaction solution was cooled to a reation vessel temperature of 50°C or below. DMF was removed by an evaporator, and 1 ml of distilled water was added to the residue to prepare a dispersion liquid of polyethylene glycol-bound titanium dioxide nanoparticles. Further, the dispersion liquid was subjected to HPLC [AKTA Purifier (manufactured by GE Healthcare Bioscience), column: HiPrep 16/60 Sephacryl S-300HR (manufactured by GE Healthcare Bioscience), mobile phase: phosphate buffer solution (pH 7.4), flow rate: 0.3 ml/min]. As a result, an UV absorption peak was observed in a fraction passed through the column, and this fraction was recovered. The dispersion liquid was diluted with distilled water to prepare a 0.05% (wt/vol) aqueous solution, and the diluted solution was allowed to stand for 72 hr. The dispersed particle diameter and zeta potential were measured by a dynamic light scattering method. This measurement was carried out with Zetasizer Nano ZS by charging 0.75 ml of the dispersion liquid of titanium oxide composite particles in a zeta potential measuring cell, setting various parameters of the solvent to the same values as water, and conducting the measurement at 25°C. As a result of a cumulant analysis, it was found that the dispersed particle diameter and the zeta potential were 49.5 nm and 0.196 mV, respectively.

### Example 26: Bonding of ¹⁴C-labeled catechol to titanium oxide composite particles

The titanium oxide composite particles prepared in Example 25 were dispersed in ultrapure water to a solid content of 1%. Next, ¹⁴C-labeled catechol was regulated with ultrapure water to a molar concentration of 10 mM. The 1% titanium oxide composite particle solution was mixed with an equal amount of the ¹⁴C-labeled catechol solution, and the concentration of the mixture was regulated with a PBS buffer solution (phosphate buffer physiological saline, PBS; pH 7.4) so that the final concentration of the titanium oxide composite particles and the final concentration of the ¹⁴C-labeled catechol solution each corresponded to ten-fold dilution. The solution thus prepared was transferred to a thermostatic chamber set to 40°C, and a bonding reaction was allowed to proceed for 3 hr. For the solution after the reaction, an absorption spectrum at wavelengths in the visible light region was confirmed with an ultraviolet-visible spectrophotometer. As a result, for the solution, an increase in absorbance was observed, suggesting bonding of the ¹⁴C-labeled catechol.

Further, this solution (2.5 ml) was subjected to buffer exchange with a free-fall column PD-10 (manufactured by GE Healthcare Bioscience) using 3.5 ml of water to remove catechol remaining unreacted. The solution thus collected was diluted with ultrapure water to a solid content of 0.01% by weight, and the diluted solution was used for the measurement of the average dispersed particle diameter with Zetasizer Nano ZS (manufactured by SYSMEX CORPORATION) in the same manner as in Example 12. As a result, the dispersed particle diameter was 35.7 nm, indicating that ¹⁴C-labeled catechol-bound titanium oxide composite particles were produced.

### Example 27: Animal test on retention in blood and accumulation in tumor

The ¹⁴C-labeled catechol-bound titanium oxide composite particles produced in Example 26 were diluted with a PBS buffer solution (phosphate buffer physiological saline, PBS; pH 7.4) to a solid content of 0.05% by weight to prepare a test solution. Human bladder cancer-derived cells T-24 were inoculated (2.5 × 10⁶ cells, 50 µl into the back of nude mice (BALB/c) to form tumor. Thus, tumor mice (9 to 10 weeks old) were provided. The test solution (100 µl was administered into a caudal vein with a syringe. After the administration, the harvesting of the organ and blood collection were carried out over time to provide measuring samples. The measuring samples were carbonated and were then used for radioactivity measurement by an accelerator mass analysis. As a result, the concentration ratio between 8 hr after the administration and 48 hr after the administrtion was 0.29, indicating that the retention in blood was high. Further, as shown in Table 2, the ratio of the concentration in the tumor to the concentration in normal cells (muscle), that is, T/N ratio, was 2.56 24 hr after the administration, confirming that the tumor accumulation was high.

**[Table 2]**

| Time (hr) | Radiation dose per tissue weight (dpm/g) | | |
|---|---|---|---|
| | Muscle | Tumor | T/N ratio |
| 24 | 31.608 | 80.762 | 2.56 |

### Example 28: Bonding of catechol to titanium oxide composite particles

The titanium oxide composite particles prepared in Example 25 were diluted with ultrapure water to a solid content of 1% by weight. Next, catechol was regulated with ultrapure water to a molar concentration of 10 mM. The 1 wt% titanium oxide composite particle solution was mixed with an equal amount of the catechol solution, and the concentration of the mixture was regulated with a PBS buffer solution (phosphate buffer physiological saline, PBS; pH 7.4) so that the final concentration of the titanium oxide composite particles and the final concentration of the catechol solution each corresponded to ten-fold dilution. The solution thus prepared was transferred to a thermostatic chamber set to 40°C, and a bonding reaction was allowed to proceed for 3 hr. For the solution after the reaction, an absorption spectrum at wavelengths in the visible light region was confirmed with an ultraviolet-visible spectrophotometer. As a result, for the solution, an increase in absorbance was observed, suggesting bonding of the catechol.

Further, this solution (2.5 ml) was subjected to buffer exchange with a free-fall column PD-10 (manufactured by GE Healthcare Bioscience) using 3.5 ml of water to remove catechol remaining unreacted and to collect the solution. These procedures revealed the preparation of catechol-bound titanium oxide composite particles.

### Example 29: Introduction of 4-aminosalicylic acid-bound polyethylene glycol into titanium dioxide particles

Polyethylene glycol modified with an isocyanate group at one terminal (average molecular weight; 20000, manufactured by SUNBIO) and 4-aminosalicylic acid (molecular weight Mn = 153.14: MP Biomedicals, Inc.) were each regulated with dimethylformamide to a final concentration of 3 mM to provide 2 ml of a reaction solution. A thermal reaction of this reaction solution was allowed to proceed at 70°C for 24 hr. The dimethylformamide was removed from the solution by an evaporator. After the removal of the dimethylformamide was confirmed, 20 ml of ultrapure water was added to prepare an aqueous solution. The aqueous solution thus prepared was transferred to a Spectra/Pore CE dialysis tube (cut-off molecular weight = 3500, manufactured by Spectrum Laboratories, Inc.) as a dialytic membrane and was dialyzed against 4 liters of ultrapure water at room temperature for 24 hr. After the dialysis, the whole solution was transferred to an egg-plant type flask and was lyophilized overnight, and 2.5 ml of dimethylformamide (DMF: manufactured by Wako Pure Chemical Industries, Ltd.) was added to and mixed with the resultant powder to prepare a 4-aminosalicylic acid-bound polyethylene glycol solution.

The 4-aminosalicylic acid-bound polyethylene glycol solution was mixed with an equal amount of a solution containing anatase-type titanium dioxide sol, prepared in Example 12, regulated with dimethylformamide to a solid content of 2% to prepare a 2.5-ml reaction solution. This reaction solution was transferred to a hydrothermal reaction vessel (HU-50, manufactured by SAN-Al Science Co. Ltd.), and a thermal reaction was allowed to proceed at 150°C for 16 hr. After the completion of the reaction, the reaction solution was cooled to a reaction vessel temperature of 50°C or below. DMF was removed by an evaporator, and 1 ml of distilled water was added to the residue to prepare a dispersion liquid of polyethylene glycol-bound titanium dioxide nanoparticles. Further, the dispersion liquid was subjected to HPLC [AKTA Purifier (manufactured by GE Healthcare Bioscience), column: HiPrep 16/60 Sephacryl S-300HR (manufactured by GE Healthcare Bioscience), mobile phase: phosphate buffer solution (pH 7.4), and flow rate: 0.3 ml/min]. As a result, an UV absorption peak was observed in a fraction passed through the column, and this fraction was collected. The dispersion liquid was diluted with distilled water to prepare a 0.05% (wt/vol) aqueous solution, and the diluted solution was allowed to stand for one hr. The dispersed particle diameter and zeta potential were measured by a dynamic light scattering method. This measurement was carried out with Zetasizer Nano ZS by charging 0.75 ml of the dispersion liquid of polyethylene glycol-bound titanium dioxide nanoparticles in a zeta potential measuring cell, setting various parameters of the solvent to the same values as water, and conducting the measurement at 25°C. As a result of a cumulant analysis, it was found that the dispersed particle diameter was 152 nm.

### Example 30: Test on single administration regarding safety

The titanium oxide composite particles prepared in Example 25 were regulated with a PBS buffer solution (phosphate buffer physiological saline, PBS; pH 7.4) to solid contents of 1% by weight, 0.5% by weight and 0.05% by weight to prepare test solutions. For each of the test solutions having respective concentrations, five nude mice (BALB/c) were provided. For each nude mouse, 100 µl of the test solution was administered through the caudal vein with a syringe, and, 24 hr after the administration, it was found that any mouse was not dead. The results demonstrate that, in the single administration test, the titanium oxide composite particles were safe.

## Claims

1. Titanium oxide composite particles comprising:
titanium oxide particles; and
a nonionic hydrophilic polymer bound to a surface of the titanium oxide particles through at least one functional group selected from carboxyl group, amino group, diol group, salicylic acid group, and phosphoric acid group,
wherein the nonionic hydrophilic polymer is polyethylene glycol, and the bonding amount of the hydrophilic polymer per unit weight of the titanium oxide composite particles is 0.3 to 0.5 g/g.

2. The titanium oxide composite particles according to claim 1, wherein the functional group is provided by a carboxylic acid or an amine, and at least an end of the hydrophilic polymer is modified with the carboxylic acid or the amine.

3. The titanium oxide composite particles according to any one of claim 1 or 2, wherein the functional group is provided by the carboxylic acid or the amine, and the carboxylic acid or the amine gets together with the hydrophilic polymer to form a copolymer.

4. The titanium oxide composite particles according to claim 1, wherein the functional group is provided by a polycarboxylic acid as a linker.

5. The titanium oxide composite particles according to claim 1, wherein the functional group is provided by a polyamine as a linker.

6. The titanium oxide composite particles according to any one of claims 1 to 5, which further comprise a ligand molecule containing the functional group and wherein the hydrophilic polymer is bound to the surface of the titanium oxide particles through the ligand molecule.

7. The titanium oxide composite particles according to claim 6, wherein the ligand molecule is at least one member selected from the group consisting of protocatechuic acid, gallic acid, methyldopa, 4-aminosalicylic acid, and quinic acid.

8. The titanium oxide composite particles according to any one of claims 1 to 7, which have a zeta potential of -20 to +20 mV.

9. The titanium oxide composite particles according to any one of claims 1 to 8, which have a diameter of 20 to 200 nm.

10. The titanium oxide composite particles according to any one of claims 1 to 9, which are rendered cytotoxic upon ultrasonic irradiation.

11. A dispersion liquid comprising the titanium oxide composite particles according to any one of claims 1 to 10 and a solvent for dispersing the particles.

12. The dispersion liquid according to claim 11, wherein the solvent is an aqueous solvent.

## Patentansprüche

1. Titanoxidverbundteilchen, umfassend:
Titanoxidteilchen; und
ein nichtionisches hydrophiles Polymer, welches durch mindestens eine Funktionsgruppe an eine Fläche der Titanoxidteilchen gebunden ist, die aus der Carboxylgruppe, der Amigogruppe, der Diolgruppe, der Salicylsäuregruppe und der Phosphorsäuregruppe gewählt wird,
wobei das nichtionische hydrophile Polymer Polyethylenglykol ist und die Bindungsmenge des hydrophilen Polymers pro Einheitsgewicht der Titanoxidverbundteilchen 0,3 bis 0,5 g/g beträgt.

2. Titanoxidverbundteilchen nach Anspruch 1, wobei die Funktionsgruppe von einer Carbonsäure oder einem Amin bereitgestellt wird und mindestens ein Ende des hydrophilen Polymers mit der Carbonsäure oder dem Amin modifiziert ist.

3. Titanoxidverbundteilchen nach einem der Ansprüche 1 oder 2, wobei die Funktionsgruppe durch die Carbonsäure oder das Amin bereitgestellt wird und die Carbonsäure oder das Amin mit dem hydrophilen Polymer zusammenkommt, um ein Copolymer zu bilden.

4. Titanoxidverbundteilchen nach Anspruch 1, wobei die Funktionsgruppe durch eine Polycarbonsäure als Linker bereitgestellt wird.

5. Titanoxidverbundteilchen nach Anspruch 1, wobei die Funktionsgruppe durch ein Polyamin als Linker bereitgestellt wird.

6. Titanoxidverbundteilchen nach einem der Ansprüche 1 bis 5, die des Weiteren ein Ligandenmolekül umfassen, welches die Funktionsgruppe enthält, und wobei das hydrophile Polymer durch das Ligandenmolekül an die Fläche der Titanoxidteilchen gebunden ist.

7. Titanoxidverbundteilchen nach Anspruch 6, wobei das Ligandenmolekül mindestens ein Element ist, welches aus der Gruppe gewählt wird, die aus Protocatechusäure, Gallussäure, Methyldopa, 4-Aminosalicylsäure und Chinasäure besteht.

8. Titanoxidverbundteilchen nach einem der Ansprüche 1 bis 7, die ein Zetapotential von -20 bis +20 mV aufweisen.

9. Titanoxidverbundteilchen nach einem der Ansprüche 1 bis 8, die einen Durchmesser von 20 bis 200 nm aufweisen.

10. Titanoxidverbundteilchen nach einem der Ansprüche 1 bis 9, die durch Ultraschallbestrahlung zytotoxisch werden.

11. Dispersionsflüssigkeit, umfassend die Titanoxidverbundteilchen nach einem der Ansprüche 1 bis 10 und ein Lösungsmittel, um die Teilchen zu lösen.

12. Dispersionsflüssigkeit nach Anspruch 11, wobei das Lösungsmittel ein wässriges Lösungsmittel ist.

## Revendications

1. Particules composites d'oxyde de titane comprenant :
des particules d'oxyde de titane ; et
un polymère hydrophile non ionique lié à une surface des particules d'oxyde de titane par l'intermédiaire d'au moins un groupe fonctionnel choisi parmi un groupe carboxyle, un groupe amino, un groupe diol, un groupe acide salicylique et un groupe acide phosphorique,
le polymère hydrophile non ionique étant du polyéthylène glycol, et la quantité de liaison du polymère hydrophile par poids unitaire des particules composites d'oxyde de titane allant de 0,3 à 0,5 g/g.

2. Particules composites d'oxyde de titane selon la revendication 1, le groupe fonctionnel étant apporté par un acide carboxylique ou une amine, et une extrémité au moins du polymère hydrophile étant modifié au moyen de l'acide carboxylique ou de l'amine.

3. Particules composites d'oxyde de titane selon l'une quelconque des revendications 1 ou 2, le groupe fonctionnel étant apporté par l'acide carboxylique ou l'amine, et l'acide carboxylique ou l'amine s'assemblant avec le polymère hydrophile pour former un copolymère.

4. Particules composites d'oxyde de titane selon la revendication 1, le groupe fonctionnel étant apporté par un acide polycarboxylique en tant que jonction.

5. Particules composites d'oxyde de titane selon la revendication 1, le groupe fonctionnel étant apporté par une polyamine en tant que jonction.

6. Particules composites d'oxyde de titane selon l'une quelconque des revendications 1 à 5, comprenant en outre une molécule de ligand contenant le groupe fonctionnel, et dans lesquelles le polymère hydrophile est lié à la surface des particules d'oxyde de titane par le biais de la molécule de ligand.

7. Particules composites d'oxyde de titane selon la revendication 6, la molécule de ligand étant au moins un élément choisi dans le groupe comprenant l'acide protocatéchique, l'acide gallique, la méthyldopa, l'acide 4-aminosalicylique et l'acide quinique.

8. Particules composites d'oxyde de titane selon l'une quelconque des revendications 1 à 7, possédant un potentiel zêta de -20 à +20 mV.

9. Particules composites d'oxyde de titane selon l'une quelconque des revendications 1 à 8, ayant un diamètre de 20 à 200 nm.

10. Particules composites d'oxyde de titane selon l'une quelconque des revendications 1 à 9, que l'on rend cytotoxiques par irradiation aux ultrasons.

11. Liquide de dispersion comprenant les particules composites d'oxyde de titane selon l'une quelconque des revendications 1 à 10 et un solvant pour disperser les particules.

12. Liquide de dispersion selon la revendication 11, le solvant étant un solvant aqueux.
